# EUROPEAN PATENT APPLICATION

(11) **EP 2 492 341 A1**
(43) Date of publication of application: **29.08.2012**
(21) Application number: 10824506.9
(22) Date of filing: 22.10.2010
(51) Int. Cl.: C12N 5/0775, A61K 35/28, A61P 7/00

(54) **MULTIPOTENT NESTIN-POSITIVE CELLS**

(30) Priority: 22.10.2009 ES 200930890
(71) Applicant: Centro Nacional de Investigaciones Cardiovasculares (CNIC), 28029 Madrid (ES); Hospital Clínic de Barcelona, 08036 Barcelona (ES); Servicio Andaluz De Salud, 41071 Sevilla (ES)
(72) Inventor: MÉNDEZ FERRER, Simón, E-41012 Sevilla (ES)
(74) Representative: Pons Ariño, Angel
(86) International application number: PCT/ES2010/070682
(87) International publication number: WO 2011/048253

(57) **Abstract**

The present invention relates to the use of at least one isolated multipotent stem cell for maintaining haematopoiesis in vitro, in which said multipotent stem cell is preferably a mesenchymal stem cell or, more preferably, said mesenchymal stem cell is a mesenchymal stem cell capable of expressing the nestin protein. The present invention also relates to an isolated cell population of adult nestin-positive mesenchymal cells from a mammal, including humans, to the use thereof for producing a drug for maintaining haematopoiesis in a mammal, for the prevention and/or treatment of at least one disease associated with a malfunction in maintaining haematopoiesis in a mammal, and for maintaining and expanding adult haematopoietic stem cells of said mammal, including a human. Furthermore, the present invention also relates to a method for maintaining haematopoiesis in vitro or to a method for evaluating the haematopoietic capacity of a mammal.

LISTADO DE SECUENCIAS

<110>  FUNDACION PÚBLICA ANDALUZA PARA LA GESTIÓN DE LA
       INVESTIGACIÓN EN SALUD EN SEVILLA
       Méndez Ferrer, Simón

<120>  Uso de células mesenquimales Nestina positivas para el
       mantenimeinto de la hematopoyesis

<130>  1985.17

<160>  42

<170>  PatentIn version 3.5

<210>  1
<211>  17
<212>  DNA
<213>  Mus musculus

<400>  1
cgccaaggtc gtcgccg                                                    17


<210>  2
<211>  19
<212>  DNA
<213>  Mus musculus

<400>  2
ttggctctgg cgatgtggc                                                  19


<210>  3
<211>  24
<212>  DNA
<213>  Mus musculus

<400>  3
atcatggccg acaagcagaa gaac                                            24


<210>  4
<211>  24
<212>  DNA
<213>  Mus musculus

<400>  4
gtacagctcg tccatgccga gagt                                            24


<210>  5
<211>  19
<212>  DNA
<213>  Mus musculus

<400>  5
ccctgaagac tcgggccta                                                  19


<210>  6
<211>  23
<212>  DNA
<213>  Mus musculus

<400>  6
caattacaag cgaaatgaga gcc                                              23


<210>  7
<211>  23
<212>  DNA
<213>  Mus musculus

<400>  7
ctcgtcagac attcatcatc cag                                              23


<210>  8
<211>  22
<212>  DNA
<213>  Mus musculus

<400>  8
caccttcttt agtgcaaagg ct                                               22


<210>  9
<211>  20
<212>  DNA
<213>  Mus musculus

<400>  9
gtgctgctcg caagttgaag                                                  20


<210>  10
<211>  21
<212>  DNA
<213>  Mus musculus

<400>  10
agttcaccag tgtttgtgtg c                                                21


<210>  11
<211>  22
<212>  DNA
<213>  Mus musculus

<400>  11
gacctgttcc agcgagggtc ta                                               22


<210>  12
<211>  26
<212>  DNA
<213>  Mus musculus

<400>  12
cttccatcct catagcaatt aaggtg                                           26


<210>  13
<211>  22
<212>  DNA
<213>  Mus musculus

<400>  13
tccctcgatg tcatccctgt tg                                               22

<210>  14
<211>  22
<212>  DNA
<213>  Mus musculus

<400>  14
ggcactctcc tggctctctt tg                                                22


<210>  15
<211>  22
<212>  DNA
<213>  Mus musculus

<400>  15
accataacga cctggaatct gt                                                22


<210>  16
<211>  21
<212>  DNA
<213>  Mus musculus

<400>  16
aacgagtgtc attagccttg c                                                 21


<210>  17
<211>  25
<212>  DNA
<213>  Mus musculus

<400>  17
cacaatatca aggatatcga cgtga                                             25


<210>  18
<211>  25
<212>  DNA
<213>  Mus musculus

<400>  18
acatcagttc tgttcttcgg gtaca                                             25


<210>  19
<211>  21
<212>  DNA
<213>  Mus musculus

<400>  19
ccccaagcac agacttttga g                                                 21


<210>  20
<211>  20
<212>  DNA
<213>  Mus musculus

<400>  20
gctttctgca tctccagcct                                                   20

<210> 21
<211> 20
<212> DNA
<213> Mus musculus

<400> 21
ttacctacac cccgccagtc 20


<210> 22
<211> 19
<212> DNA
<213> Mus musculus

<400> 22
tgctggtctg gaagggtcc 19


<210> 23
<211> 20
<212> DNA
<213> Mus musculus

<400> 23
atggcgtcct ctctgcttga 20


<210> 24
<211> 20
<212> DNA
<213> Mus musculus

<400> 24
gaagggtggg tagtcatttg 20


<210> 25
<211> 21
<212> DNA
<213> Mus musculus

<400> 25
gggcaataag gtagtgaaca g 21


<210> 26
<211> 21
<212> DNA
<213> Mus musculus

<400> 26
gcagcacagg tcctaaatag t 21


<210> 27
<211> 20
<212> DNA
<213> Mus musculus

<400> 27
taagaactgc cgtcgccatt 20


<210> 28
<211> 20

<212>  DNA
<213>  Mus musculus

<400>  28
ggccacaatc caatcattcc                                                    20


<210>  29
<211>  20
<212>  DNA
<213>  Mus musculus

<400>  29
accactcgca ttcctttgac                                                    20


<210>  30
<211>  20
<212>  DNA
<213>  Mus musculus

<400>  30
tgggtcagct cttgtgaatg                                                    20


<210>  31
<211>  24
<212>  DNA
<213>  Mus musculus

<400>  31
tgcatcaact cagagtgtca atca                                               24


<210>  32
<211>  19
<212>  DNA
<213>  Mus musculus

<400>  32
tgcgcagatt gcaggttgt                                                     19


<210>  33
<211>  21
<212>  DNA
<213>  Mus musculus

<400>  33
cacgctacac cctggacttt g                                                  21


<210>  34
<211>  21
<212>  DNA
<213>  Mus musculus

<400>  34
ccatctcctc agcgaagcag t                                                  21


<210>  35
<211>  17
<212>  DNA
<213>  Mus musculus

<400>  35
caagcggagg ccgaaga                                                    17

<210>  36
<211>  18
<212>  DNA
<213>  Mus musculus

<400>  36
cagcttgcac gtcggttt                                                   18

<210>  37
<211>  23
<212>  DNA
<213>  Mus musculus

<400>  37
agatccacaa ccattcgaca ttt                                             23

<210>  38
<211>  21
<212>  DNA
<213>  Mus musculus

<400>  38
tcccaggtac atcacagagg g                                               21

<210>  39
<211>  21
<212>  DNA
<213>  Mus musculus

<400>  39
agaacacggc aaggtgaaga t                                               21

<210>  40
<211>  23
<212>  DNA
<213>  Mus musculus

<400>  40
tgaacccata gatgtaccac tgg                                             23

<210>  41
<211>  20
<212>  DNA
<213>  Mus musculus

<400>  41
tgtgtccgtc gtggatctga                                                 20

<210>  42
<211>  21
<212>  DNA
<213>  Mus musculus

<400>  42

cctgcttcac caccttcttg a 21

LISTADO DE SECUENCIAS

<110> CENTRO NACIONAL DE INVESTIGACIONES CARDIOVASCULARES (CNIC)
 HOSPITAL CLÍNIC DE BARCELONA
 SERVICIO ANDALUZ DE SALUD

<120> MULTIPOTENT NESTIN-POSITIVE CELLS

<130> 1985.17

<160> 42

<170> PatentIn version 3.5

<210> 1
<211> 17
<212> DNA
<213> Mus musculus

<400> 1
cgccaaggtc gtcgccg 17

<210> 2
<211> 19
<212> DNA
<213> Mus musculus

<400> 2
ttggctctgg cgatgtggc 19

<210> 3
<211> 24
<212> DNA
<213> Mus musculus

<400> 3
atcatggccg acaagcagaa gaac 24

<210> 4
<211> 24
<212> DNA
<213> Mus musculus

<400> 4
gtacagctcg tccatgccga gagt 24

<210> 5
<211> 19
<212> DNA
<213> Mus musculus

<400> 5
ccctgaagac tcgggccta 19

<210> 6
<211> 23
<212> DNA
<213> Mus musculus

<400>  6
caattacaag cgaaatgaga gcc                                              23


<210>  7
<211>  23
<212>  DNA
<213>  Mus musculus

<400>  7
ctcgtcagac attcatcatc cag                                              23


<210>  8
<211>  22
<212>  DNA
<213>  Mus musculus

<400>  8
caccttcttt agtgcaaagg ct                                               22


<210>  9
<211>  20
<212>  DNA
<213>  Mus musculus

<400>  9
gtgctgctcg caagttgaag                                                  20


<210>  10
<211>  21
<212>  DNA
<213>  Mus musculus

<400>  10
agttcaccag tgtttgtgtg c                                                21


<210>  11
<211>  22
<212>  DNA
<213>  Mus musculus

<400>  11
gacctgttcc agcgagggtc ta                                               22


<210>  12
<211>  26
<212>  DNA
<213>  Mus musculus

<400>  12
cttccatcct catagcaatt aaggtg                                           26


<210>  13
<211>  22
<212>  DNA
<213>  Mus musculus

<400>  13
tccctcgatg tcatccctgt tg                                               22

<210>  14
<211>  22
<212>  DNA
<213>  Mus musculus

<400>  14
ggcactctcc tggctctctt tg                                                22


<210>  15
<211>  22
<212>  DNA
<213>  Mus musculus

<400>  15
accataacga cctggaatct gt                                                22


<210>  16
<211>  21
<212>  DNA
<213>  Mus musculus

<400>  16
aacgagtgtc attagccttg c                                                 21


<210>  17
<211>  25
<212>  DNA
<213>  Mus musculus

<400>  17
cacaatatca aggatatcga cgtga                                             25


<210>  18
<211>  25
<212>  DNA
<213>  Mus musculus

<400>  18
acatcagttc tgttcttcgg gtaca                                             25


<210>  19
<211>  21
<212>  DNA
<213>  Mus musculus

<400>  19
ccccaagcac agacttttga g                                                 21


<210>  20
<211>  20
<212>  DNA
<213>  Mus musculus

<400>  20
gctttctgca tctccagcct                                                   20

<210> 21
<211> 20
<212> DNA
<213> Mus musculus

<400> 21
ttacctacac cccgccagtc 20


<210> 22
<211> 19
<212> DNA
<213> Mus musculus

<400> 22
tgctggtctg gaagggtcc 19


<210> 23
<211> 20
<212> DNA
<213> Mus musculus

<400> 23
atggcgtcct ctctgcttga 20


<210> 24
<211> 20
<212> DNA
<213> Mus musculus

<400> 24
gaagggtggg tagtcatttg 20


<210> 25
<211> 21
<212> DNA
<213> Mus musculus

<400> 25
gggcaataag gtagtgaaca g 21


<210> 26
<211> 21
<212> DNA
<213> Mus musculus

<400> 26
gcagcacagg tcctaaatag t 21


<210> 27
<211> 20
<212> DNA
<213> Mus musculus

<400> 27
taagaactgc cgtcgccatt 20


<210> 28
<211> 20

<212>  DNA
<213>  Mus musculus

<400>  28
ggccacaatc caatcattcc                                                    20


<210>  29
<211>  20
<212>  DNA
<213>  Mus musculus

<400>  29
accactcgca ttcctttgac                                                    20


<210>  30
<211>  20
<212>  DNA
<213>  Mus musculus

<400>  30
tgggtcagct cttgtgaatg                                                    20


<210>  31
<211>  24
<212>  DNA
<213>  Mus musculus

<400>  31
tgcatcaact cagagtgtca atca                                               24


<210>  32
<211>  19
<212>  DNA
<213>  Mus musculus

<400>  32
tgcgcagatt gcaggttgt                                                     19


<210>  33
<211>  21
<212>  DNA
<213>  Mus musculus

<400>  33
cacgctacac cctggacttt g                                                  21


<210>  34
<211>  21
<212>  DNA
<213>  Mus musculus

<400>  34
ccatctcctc agcgaagcag t                                                  21


<210>  35
<211>  17
<212>  DNA
<213>  Mus musculus

<400>  35
caagcggagg ccgaaga                                                    17

<210>  36
<211>  18
<212>  DNA
<213>  Mus musculus

<400>  36
cagcttgcac gtcggttt                                                   18

<210>  37
<211>  23
<212>  DNA
<213>  Mus musculus

<400>  37
agatccacaa ccattcgaca ttt                                             23

<210>  38
<211>  21
<212>  DNA
<213>  Mus musculus

<400>  38
tcccaggtac atcacagagg g                                               21

<210>  39
<211>  21
<212>  DNA
<213>  Mus musculus

<400>  39
agaacacggc aaggtgaaga t                                               21

<210>  40
<211>  23
<212>  DNA
<213>  Mus musculus

<400>  40
tgaacccata gatgtaccac tgg                                             23

<210>  41
<211>  20
<212>  DNA
<213>  Mus musculus

<400>  41
tgtgtccgtc gtggatctga                                                 20

<210>  42
<211>  21
<212>  DNA
<213>  Mus musculus

<400>  42

cctgcttcac caccttcttg a 21

## Description

The present invention relates to the use of at least one isolated multipotential stem cell for the maintenance of haematopoiesis *in vitro,* where, preferably, said multipotential stem cell is a mesenchymal stem cell or, more preferably, said mesenchymal stem cell is a mesenchymal cell capable of expressing the Nestin protein. The present invention also relates to an isolated cell population of adult nestin-positive mesenchymal cells of a mammal, including human cells, to the use thereof for the manufacturing of a medicament designed for the maintenance of haematopoiesis in a mammal, for the prevention and/or treatment of at least one disease associated with a dysfunction of the maintenance of haematopoiesis in a mammal, and for maintaining and expanding the adult haematopoietic stem cells of said mammal, including a human being. Moreover, the present invention also relates to a method for maintaining haematopoiesis *in vitro* or to a method for evaluating the haematopoietic capacity of a mammal.

### PRIOR STATE OF THE ART

It has been suggested that haematopoietic stem cells (HSCs) are preferably located in perivascular regions of the bone marrow (Kiel et al., 2005. Cell 121: 1109-1121), close to reticular cells with a high expression of the CXCL12/SDF-1 chemokine (Sugiyama et al., 2006. Immunity 25: 977-988). However, the identity and the function of these cells are unknown. In human bone marrow, CD146 is expressed in perivascular cells that have osteoprogenitor capacity and may self-renew and reconstitute haematopoiesis (Sacchetti et al., 2007. Cell 131: 324-336). However, it is not clear whether CD45- CD146⁺ cells are a part of the haematopoietic niche and this combination of markers is also present in vascular endothelial cells.

The migration of HSCs offers a useful paradigm to study the cellular elements that form the haemopoietic niche, since the attraction of HSCs is directly controlled by the bone marrow microenvironment. Previous studies have revealed that the mobilisation of HSCs induced by the granulocyte colony-stimulating factor (G-CSF) is regulated by the sympathetic nervous system (SNS) (Katayama et al., 2006. Cell, 124: 407-421). In homeostasis, a low number of HSCs abandon the bone marrow and go into the blood stream (Abkowitz et al., 2003. Blood, 102: 1249-1253; Wright et al., 2001. Science, 294: 1933-1936). The release thereof into the blood is regulated by the molecular clock and directed by the SNS, which cyclically releases noradrenaline in the bone marrow, thereby activating the β₃ adrenergic receptors and consequently inducing rhythmic oscillations in the expression of *Cxc*/*12* (Mendez-Ferrer et al., 2008. Nature, 452: 442-447).

On the other hand, previous studies have shown that the CD45⁻ CD146⁺ perivascular cells in the bone marrow and other tissues contain osteoprogenitors (Sacchetti et al., 2007. Cell 131: 324-336) or MSCs (Crisan et al., 2008. Cell Stem Cell, 3: 301-313), and recent data suggest that cells capable of endochondral ossification are necessary for the formation of the haemopoietic niche in foetal bone marrow (Chan et al., 2008. Nature, 457[7228]: 490-4).

Nestin is an intermediate filament protein. It is a marker of multi-lineage progenitor cells and its presence in these cells indicates the multipotentiality of the cells and their regenerative capacity (Wiese et al., 2004. CMLS, Cell. Mol. Life Sci., 61: 2510-2522). In this regard, it is well-known that Nestin+ cells in the bulb region of the capillary follicle have stem cell properties, are multipotent (mesenchymal stem cells) and may generate neural lineage cells *in vitro* and *in vivo* (Mignone et al., 2007. Cell Cycle, 6 (17): 2161-2170). The discussion in Mignore et al., 2007, mentions the cell types whereinto Nestin+ cells may differentiate, for example, neural stem cells and neuronal cells, hepatic oval cells, muscle satellite cells, pancreatic stem cells, Leydig progenitor cells, smooth muscle cells, sebaceous gland cells, melanocytes and keratinocytes.

The transplantation of haematopoietic progenitor cells involves the infusion of these cells obtained from the bone marrow, the peripheral blood (Körbling et al., 1981. Exp Hematol 9: 684-690), the umbilical cord or the foetal liver into a patient who has previously been conditioned to receive the graft. It has become a therapeutic method for a wide variety of diseases, such as malignant haemopathies, aplastic anaemia, immunodeficiencies and a large number of solid tumours. Currently, over 30,000 patients are transplanted every year worldwide. The selection of the source and the type of transplant are determined by different factors. Peripheral blood (PB) was the source of haematopoietic progenitors in 90% of autologous transplants and in 30% of allogeneic transplants.

The morbidity and mortality of the procedure have improved in recent years thanks to a better knowledge of the histocompatibility system, the development of anti-infective therapy, the use of environments with scant microbial contamination, haemotherapeutic support and the administration of potent immunosuppressants.

It is necessary to guarantee a correct haematopoietic function following high-dose chemoradiotherapeutic treatment. Moreover, there are several limitations to allogeneic transplantation, which has led to the development of autotransplantation in recent years. This involves obtaining haemotopoietic progenitor cells from the patients themselves, preserving them and reinfusing them, after administering doses of ablative chemotherapy and/or radiotherapy.

However, despite the fact that autologous transplants have a number of advantages (they do not require searching for a donour, lower toxicity related to the procedure, they may be performed on older patients (60 - 65 years old), there are no undesirable graft-versus-host disease (GVHD) complications, they allow for consolidation in solid tumours, when the tumoral mass is smaller, a lower number of cells are required for medullary reconstruction than in allogeneic transplants, and it is an option for patients who, for various reasons, cannot receive allogeneic transplants), they present a lower frequency of medullary recovery due to defects in the medullary microenvironment, influenced by the baseline disease and/or previous treatments.

The transplantation of haematopoietic precursors obtained from peripheral blood requires the administration of granulo-monocytic CSF (GM-CSF). The main advantage of HTC from PB over bone marrow transplantation lies in the fact that the former contain a greater number of cells and better implantation is achieved. Moreover, it presents a number of additional advantages: there is no need to use an operating-room, which prevents all the risks that this entails, fast haematological recovery following the implantation, lower possibility of obtaining malignant cells, reduced risk of infection of the collected material, and haematopoietic cells may be obtained in situations wherein it is difficult to extract them from the bone marrow (myelofibrosis, pelvic irradiation, etc.)

However, sometimes the necessary cellularity is not obtained with a single apheresis process, and several donation (or self-donation) processes are necessary, with the consequent damage to the donour, who suffers various symptoms: lumbar, bone and muscle pain, cephalea, hypotension, general discomfort, somnolence, loss of appetite, rashes, nausea, fever, fluid retention, etc. Cases of cardiac arrest and myocardial infarction during the apheresis have been described in donours with a history of cardiovascular diseases. Other complications include increased size of the spleen and splenic rupture, as well as signs of extramedullary haematopoiesis. Epiescleritis and iritis may also appear.

Currently, the umbilical cord is the third source of cells for transplantation in adults and the second in children. It has been used in genetic and malignant diseases and in patients with total or partial compatibility, familial and nonfamilial. However, the use of immature, compromised haematopoietic progenitors obtained from the umbilical cord has the disadvantage that its efficacy in adults is yet to be proven, since the number of cells is too small to provide a lasting implantation (Broxmeyer et al., 1990. Int J Cell Cloning 8: 76).

Consequently, it is difficult to maintain the production of blood cells derived from haematopoietic stem cells (*in vivo* and/or *in vitro*), and to expand them, for example in order to apply them in therapy.

### EXPLANATION OF THE INVENTION

The present invention relates to the use of at least one isolated multipotential stem cell for the maintenance of haematopoiesis *in vitro,* where, preferably, said multipotential stem cell is a mesenchymal stem cell or, more preferably, said mesenchymal stem cell is a mesenchymal cell capable of expressing the Nestin protein. The present invention also relates to the use of any of the stem cells described above for the manufacturing of a medicament designed for the maintenance of haematopoiesis in a mammal, or the prevention and/or treatment of at least one disease associated with a dysfunction of the maintenance of haematopoiesis in a mammal. Preferably, said disease evolves with a myelopoiesis or lymphopoiesis deficiency. Moreover, the present invention also relates to a method for maintaining haematopoiesis *in vitro* or a method for evaluating the haematopoietic capacity of a mammal.

The present invention demonstrates that Nestin-positive cells are mesenchymal stem cells and, therefore, multipotent, capable of differentiating into cell lineages such as, for example, osteoblast, chondrocyte or adipocyte lineages.

Said Nestin-positive cells express high levels of molecules involved in the maintenance of haematopoietic stem cells, i.e. in haematopoiesis. The expression of *Cxc*/*12,* a chemokine involved, for example, in the migration of haematopoietic cells from the foetal liver to the bone marrow, is about 50 times greater in Nestin-positive cells than in the rest of stromal cells. The expression of other genes in charge of regulating the maintenance of haematopoietic stem cells, such as, for example *Kitl, II7* or *Vcam1,* is between 140 and 800 times greater in Nestin-positive cells than in the rest of stromal cells.

In this regard, mice wherein 90% of the Nestin+ cells were selectively eliminated present a 2-4-fold reduction in the number of haemopoietic stem cells in the bone marrow have a 90% reduction in the capacity of haemopoietic progenitors to nest in the bone marrow. Likewise, whereas the total cellularity and the number of Lin- CD48⁻ bone marrow cells in these mice were not affected, the haemopoietic progenitors and the haemopoietic stem cells were reduced by ∼ 50%. This reduction was associated with a proportional, selective increase in the spleen, without any differences being detected in the cell cycle or the frequency of apoptotic cells. Therefore, these studies indicate that haematopoietic stem cells and haemopoietic progenitors were mobilised from the bone marrow to extramedullary sites after depleting the nestin⁺ cells. The severe reductions in HSCs detected suggest that nestin⁺ cells play a central role in the maintenance of HSCs and in haematopoiesis. Therefore, placing these nestin⁺ stem cells in contact with haematopoietic stem cells leads to the maintenance of haematopoiesis. This use has a clear industrial application, for example, in the field of treating diseases associated with a dysfunction of the maintenance of haematopoiesis in a mammal or the maintenance of haematopoiesis *in vitro* in order to allow for the generation of cells derived from haematopoietic stem cells, such as, for example, blood cells.

Consequently, the authors of the present invention have isolated a population of nestin-positive mesenchymal cells, which promote the self-renewal and/or expansion of haematopoietic stem cells, both *in vitro* and *in vivo.*

Therefore, a first aspect of the invention relates to an isolated cell population, hereinafter cell population of the invention, which comprises at least one multipotential Nestin-positive stem cell. In a preferred embodiment of this aspect of the invention, the multipotential Nestin-positive stem cell is a mesenchymal cell. In another, more preferred embodiment of this aspect of the invention, the multipotential Nestin-positive stem cell is a non-adherent cell. In another, more preferred embodiment of this aspect of the invention, the multipotential Nestin-positive stem cell is obtained by means of a process which comprises:
a) Obtaining a bone marrow aspirate from a mammal,
b) Lysing the red-series cells,
c) Selecting the CD45- cells, and
d) Seeding the cells of step (c) in an adequate medium.

In an even more preferred embodiment, the mammal of step (a) is a human being.

In another preferred embodiment, the cell population of the invention further comprises at least one haematopoietic stem cell. In another, more preferred embodiment, the haematopoietic stem cell is human.

Another aspect relates to a composition, hereinafter composition of the invention, which comprises the isolated cell population of the invention. In a preferred embodiment of this aspect, the composition is a pharmaceutical composition. In another, more preferred embodiment, the composition further comprises a pharmaceutically acceptable vehicle. In another, more preferred embodiment, the composition further comprises another active principle.

Another aspect relates to the use of an isolated cell population of the invention or a pharmaceutical composition of the invention for the maintenance of haematopoiesis *in vitro.* Another aspect relates to the use of an isolated cell population of the invention or a pharmaceutical composition of the invention for the maintenance of haematopoiesis *in vivo.*

Another aspect of the invention relates to the use of an isolated cell population of the invention, or a pharmaceutical composition of the invention, for the self-renewal of haematopoietic stem cells.

Another aspect of the invention relates to the use of an isolated cell population of the invention, or a pharmaceutical composition of the invention, for the expansion of haematopoietic stem cells.

Another aspect relates to the use of an isolated cell population of the invention or a pharmaceutical composition of the invention for the preparation of a medicament.

Another aspect relates to the use of an isolated cell population of the invention or a pharmaceutical composition of the invention for the preparation of a medicament designed for the maintenance of haematopoiesis in a mammal. In a preferred embodiment of this aspect of the invention, the mammal is a human being.

Another aspect relates to the use of an isolated cell population of the invention or a pharmaceutical composition of the invention for the preparation of a medicament designed for tissue repair and regeneration. In a preferred embodiment, the tissue is blood.

Another aspect relates to the use of an isolated cell population of the invention or a pharmaceutical composition of the invention for the preparation of a medicament designed for the treatment of blood and haematopoietic organ diseases. In a preferred embodiment, the disease evolves with a myelopoiesis or lymphopoiesis deficiency.

The blood and haematopoietic organ diseases are recorded, without being limited thereto, in the third chapter of the list of ICD-10 codes (tenth version of the *International Statistical Classification of Diseases and Related Health Problems).* Amongst others, in this specification, "blood and haematopoietic organ diseases" are understood to be the following:

### Acquired

• Malignant neoplasms
   ○ Haematologic
      ■ Leukemias
         ■ Acute lymphoblastic leukemia
         ■ Acute non-lymphoblastic leukemia
         ■ Chronic lymphocytic leukemia
         ■ Chronic myeloid leukemia, in accelerated phase or blast crisis
      ■ Lymphomas
         ■ Hodgkin's disease
         ■ Non-Hodgkin's lymphoma
      ■ Myelomas
         ■ Multiple myeloma (Kahler's disease)
   ○ Solid-tumour cancers
      ■ Neuroblastoma
      ■ Desmoplastic small round cell tumours
      ■ Ewing's sarcoma
      ■ Choriocarcinoma
• Haematological diseases
   ○ Phagocytic disorders
      ■ Myelodysplasia
   ○ Anaemias
      ■ Paroxysmal nocturnal haemoglobinuria (severe aplasia)
      ■ Aplastic anaemia
         ■ Pure red-cell aplasia
   ○ Myeloproliferative disorders
      ■ Polycythemia vera
      ■ Essential thrombocytosis
      ■ Myelofibrosis
• Metabolic disorders
   ○ amyloidosis
      ■ Amyloid light-chain amyloidosis
• Environmentally-induced diseases
   ○ Radiation poisoning
• Viral diseases
   ○ HIV^{[3]}

### Congenital

• Lysosomal storage diseases
   ○ Lipidosis (lipid storage disorders)
      ■ Neuronal ceroid lipofuscinosis
         ■ Infantile neuronal ceroid lipofuscinosis (INCL, Santavuori's disease)
         ■ Jansky-Bielschowsky's disease (late infantile neuronal ceroid lipofuscinosis)
      ■ Sphyngolipidosis
         ■ Niemann-Pick's disease
         ■ Gaucher' disease
      ■ Leukodystrophies
         ■ Adrenoleukodystrophy
         ■ Metachromatic leukodystrophy
         ■ Krabbe's disease (globoid-cell leukodystrophy)
   ○ Mucopolysaccharidosis
      ■
         ■ Hurler's syndrome (MPS IH, α-L-iduronidase deficiency)
         ■ Scheie's syndrome (MPS I S)
         ■ Hurler-Scheie's syndrome (MPS I H-S)
         ■ Hunter's syndrome (MPS II, iduronidase sulfate deficiency)
         ■ Sanfilippo's syndrome (MPS III)
         ■ Morquio's syndrome (MPS IV)
         ■ Maroteaux-Lamy's syndrome (MPS VI)
         ■ Sly's syndrome (MPS VII)
   ○ Glycoproteinosis
      ■
         ■ Mucolipidosis II (I-cell disease)
         ■ Fucosidosis
         ■ Aspartylglucosaminuria
         ■ Alpha-mannosidosis
   ○ Others
      ■
         ■ Wolman's disease (acid lipase deficiency)
• Immunodeficiencies
   ○ T-cell deficiency
      ■ Ataxia telangiectasia
      ■ DiGeorge's syndrome
   ○ Combined B- and T-cell deficiencies
      ■ Severe combined immunodeficiency (SCID), all types
   ○ Well-defined syndromes
      ■ Wiskott-Aldrich's syndrome
   ○ Phagocytic disorders
      ■ Kostmann's syndrome
      ■ Shwachman-Diamond's syndrome
   ○ Immune deregulation diseases
      ■ Griscelli's syndrome, type II
   ○ Innate immune deficiencies
      ■ NF-kappa-B essential modulator deficiency (NEMO) (Inhibitor of kappa light polypeptide gene enhancer in B-cell kinase gamma deficiency)
• Haematological diseases
   ○
      ■ Haemoglobinopathies
      ■ Falciform-cell disease
      ■ β-thalassaemia major (Cooley's anaemia)
   ○ Anaemias
      ■ Aplastic anaemia
         ■ Diamond-Blackfan's anaemia
         ■ Fanconi's anaemia
   ○ Cytopenias
      ■ Amegakaryocytic thrombocytopenia
   ○ Haemophagocytic syndromes
      ■ Haemophagocytic lymphohistiocytosis (HLH)

In another preferred embodiment, the disease is selected from the list that comprises: myeloma, benign monoclonal gammopathy, hypoplasia and medullary aplasia, myelofibrosis, myelodysplastic syndrome, anaemia, polycythaemia, neutropenia, acute leukemia, chronic leukemia, lymphoma, purpura, haemophilia, or any combination thereof.

Another aspect of the invention relates to a method for obtaining haematopoietic cells *in vitro,* hereinafter method for obtaining haematopoietic cells of the invention, which comprises:
a) placing at least one isolated Nestin-positive cell in contact with at least one isolated haematopoietic stem cell, and
b) incubating the product obtained in section (a) in an adequate culture medium for the division and/or differentiation of the haematopoietic stem cell. In a preferred embodiment of this aspect of the invention, the Nestin-positive cell is isolated from the bone marrow. In another preferred embodiment, the Nestin-positive cell is a multipotent stem cell. In another preferred embodiment, the Nestin-positive cell is a mesenchymal stem cell. In another preferred embodiment, the Nestin-positive cell is a non-adherent cell. In another preferred embodiment, the Nestin-positive cell is human.

Another aspect of the invention relates to the haematopoietic cells obtainable by the method for obtaining haematopoietic cells of the invention.

Another aspect of the present invention relates to the use of at least one isolated multipotential stem cell for the maintenance of haematopoiesis *in vitro.*

Stem cells are undifferentiated cells that have the capacity to divide without losing their properties and to produce both differentiated cells and undifferentiated cells. Depending on the origin of the stem cells, we can distinguish between embryonic stem cells and adult stem cells. In the present invention, we refer to an adult stem cell or an embryonic stem cell.

The multipotential stem cell of the present invention is capable of differentiating into different cell types from the same embryonic layer (Weissman et al., 2001. Annu Rev Cell Dev Biol, 17: 387-403) and, as a consequence, into any derived adult tissue.

The term "isolated" refers to the fact that the stem cells remain outside the human or animal body.

As understood in the present invention, the term "maintenance of haematopoiesis" refers to the preservation of the haematopoiesis process, i.e. the preservation of the generation, regulation and production of cells derived from haematopoietic stem cells, as well as the division of said haematopoietic stem cells. Hereinafter, the term "haemopoiesis" may be used as synonymous with the term "haematopoiesis".

Haematopoietic stem cells may be, without being limited thereto, "long-term haematopoietic stem cells" (LT-HSC) or "short-term haematopoietic stem cells" (ST-HSC).

The cells derived from haematopoietic stem cells may be, without being limited thereto, compromised haematopoietic progenitors capable of differentiating into a myelocytic or lymphopoietic cell line, erythrocytes, platelets, granulocytes (neutrophils, basophils, eosinophils), monocytes or lymphocytes. Likewise, the cells derived from haematopoietic stem cells may be any of the precursors of erythrocytes, platelets, granulocytes, monocytes or lymphocytes.

Under *in vivo* conditions, haematopoiesis is produced in various organs or tissues depending on the stage of development of the individual or even the development of pathological conditions. Therefore, during the course of approximately the second and third weeks of embryonic development, haematopoiesis is produced in the vitelline sac. Approximately from the sixth week, haematopoietic tissue appears in the liver. From the third month of embryonic development, myeloid tissue begins to develop in the bone marrow, which is where the haematopoiesis process is primarily produced. From the eighth month of embryonic development, haematopoietic tissue appears in the spleen. In adult individuals, the haematopoietic activity is maintained in the bone marrow; however, in the course of certain pathological conditions, haematopoietic foci may be observed in some of the aforementioned organs; in this case, haematopoiesis is extramedullary.

The maintenance of haematopoiesis *in vitro* of the present invention may be performed in cells isolated from the haematopoietic tissue of any of the organs that have said tissue, such as, for example, without being limited thereto, any of the organs cited in the preceding paragraph in any stage of development of the individual.

A preferred embodiment of the present invention relates to the use wherein the multipotential stem cell is a mesenchymal cell.

Mesenchymal cells are derived from any mesenchymal tissue. Mesenchymal tissue is the tissue derived from the embryonic layer called mesoderm. The mesenchymal tissue wherefrom the mesenchymal cell of the present invention is derived is selected from the list that comprises lax connective tissue, dense connective tissue, adipose tissue, cartilaginous tissue, bone tissue, haematopoietic tissue, blood tissue or muscular tissue. Preferably, the mesenchymal cell of the present invention is derived from haematopoietic tissue. More preferably, the mesenchymal cell is derived from the bone marrow. The bone marrow is a tissue that is found, for example, without being limited thereto, inside the long bones, vertebrae, ribs, sternum, cranial bones, scapular waist or pelvis. Preferably, the bone marrow is red bone marrow, which occupies the spongy tissue of flat bones, such as, for example, without being limited thereto, the sternum, the vertebrae, the pelvis or the ribs. This type of bone marrow is the one that has the haematopoietic function. Moreover, the mesenchymal cells of the present invention may be derived from the umbilical cord.

Another preferred embodiment of the present invention relates to the use wherein the mesenchymal stem cell is a Nestin-positive cell.

The Nestin protein is a type IV intermediate filament protein. This protein is expressed in undifferentiated cells during the early stages of development of the central nervous system and the peripheral nervous system, as well as organs such as the pancreas or muscular tissue. The Nestin protein is considered to be a stem cell marker. Those cells that express said protein are cells which behave like mesenchymal cells, taking into consideration their differentiation capacity. Therefore, the Nestin-positive mesenchymal cell of the present invention refers to a mesenchymal cell capable of expressing the Nestin protein. Nestin-positive mesenchymal cells may express said protein to a variable extent depending on multifactorial conditions, such as, for example, the type of tissue wherein the mesenchymal cell is found and the stage of development of the tissue whereto it belongs, etc. The examples of the present invention show that the drastic reduction in Nestin-positive mesenchymal cells in a mouse causes a 75% reduction in the number of haematopoietic bone marrow stem cells, capable of generating haemopoietic colonies after being cultured for long periods of time.

According to another preferred embodiment, the stem cell constitutes an isolated cell population. The cell population is composed of, or comprises, any of the multipotent stem cells, mesenchymal stem cells or Nestin-positive mesenchymal cells. Said cell population may be composed of any combination of multipotent stem cells, mesenchymal stem cells or Nestin-positive mesenchymal cells, or may comprise any combination of the aforementioned cells.

Another aspect of the present invention relates to the use of at least one multipotential stem cell for the manufacturing of a medicament designed for the maintenance of haematopoiesis in a mammal.

Another aspect of the invention relates to the use of at least one multipotential stem cell for the manufacturing of a medicament designed for the prevention or treatment of at least one disease associated with a dysfunction of the maintenance of haematopoiesis in a mammal.

The term "prevention", as understood in the present invention, involves avoiding the onset of diseases that evolve with a dysfunction of the maintenance of haematopoiesis. The term "treatment", as understood in the present invention, entails combatting the effects caused by said dysfunction, in order to stabilise the individual's condition or prevent subsequent damages.

The term "dysfunction of the maintenance of haematopoiesis", as used in the present invention, refers to a quantitative alteration of haematopoiesis which causes the total or partial loss of the maintenance of haematopoiesis and, as a consequence of said dysfunction, there is a decrease in cellularity (hypocellularity) in the bone marrow of the individual with respect to a normal condition. The normal condition is that wherein said cellularity is maintained above a minimum percentage, such as, for example, without being limited thereto, above 20, 25, 30 or 35%. The hypocellularity condition does not affect the present invention. I.e. the disease associated with said dysfunction of the maintenance of haematopoiesis is a consequence of the reduction in the division and/or differentiation of haematopoietic cells, which causes a decrease in the production of each of the cells derived from haematopoietic stem cells with respect to a control production value for said cells. Said comparison may be made by determining the concentration of any type of differentiated haematopoietic cell and comparing it to the reference levels. These reference levels are the control levels. For example, the reference level of erythrocytes is 4.5.10⁶ cells/mm³ in the case of males and the reference level of leukocytes is 5000 cells/mm³. The aforementioned reference levels are minimum levels below which we would consider that there is a decrease in the production of each of the cells derived from haematopoietic stem cells.

The term "cellularity", as understood in the present invention, refers to the ratio between haematopoietic cells and adipose tissue (primary constituent cells of the bone marrow), expressed as the percentage of cells.

A preferred embodiment relates to the use wherein the disease associated with the dysfunction of the maintenance of haematopoiesis is a disease that evolves with a myelopoiesis or lymphopoiesis deficiency.

The term "deficiency", as understood in the present invention, refers to a functioning that is lower than normal, i.e. to a division and/or differentiation capacity of myelopoietic or lymphopoietic stem cells that is below the reference levels established for each type of cells derived from haematopoietic stem cells and for each sex.

Myelopoiesis is the process that leads to the generation, development and maturation of blood myeloid cells, i.e. erythrocytes, platelets, granulocytes (neutrophils, basophils, eosinophils) or monocytes. Erythrocytes are generated by a process called erythropoiesis. Platelets are generated by thrombopoiesis. Granulocytes are generated by granulopoiesis. Monocytes are generated by monopoiesis. Lymphopoiesis is the process that allows for the generation, development and maturation of lymphocytes.

The disease that evolves with a myelopoiesis or lymphopoiesis deficiency is selected from the list that comprises aplastic anaemia, pancytopenia, erythroblastopenia, erythrocytic aplasia, Fanconi's anaemia, Blackfan-Diamond's syndrome, panmyelopthisis, dyserythropoietic anaemia, dyshaematopoietic anaemia, granulocytopenia (agranulocytosis), neutropenia and lymphopenia.

Another preferred embodiment of the present invention relates to the use of at least one multipotential stem cell for the manufacturing of a medicament, where the multipotential stem cell is a mesenchymal cell. Another preferred embodiment relates to the use wherein the mesenchymal stem cell is a Nestin-positive cell. According to another preferred embodiment, the stem cell constitutes an isolated cell population.

Said medicament comprises:
- the multipotential stem cell, mesenchymal cell or Nestin-positive mesenchymal cell, and
- a pharmacologically acceptable vehicle,
- a pharmacologically acceptable excipient, or
- another active substance.

The medicament may comprise any of said cells with the vehicle, excipient or other active substance in any combination thereof.

Moreover, the vehicle and the excipient must be pharmaceutically acceptable.

The term "vehicle" refers to those substances, or combination of substances, known in the pharmaceutical sector, which are used in the preparation of pharmaceutical administration forms, and include, without being limited thereto, solids, liquids, solvents and surfactants. The vehicle may be an inert substance or a substance with an action analogous to that of any of the sequences of the present invention. The function of the vehicle is to facilitate the incorporation of the multipotential stem cell, mesenchymal cell or Nestin-positive mesenchymal cell and/or other compounds, allow for a better dosage and administration, or give consistency and shape to the medicament. When the form of presentation is liquid, the vehicle is the diluent.

The term "excipient" refers to a substance that contributes to the absorption of any of the sequences of the present invention, stabilises said sequence or contributes to the preparation of the medicament in the sense of giving it consistency or providing flavours that make it more pleasant. Thus, excipients may have the function of maintaining the ingredients bound, such as, for example, starches, sugars or celluloses; a sweetening function; a colouring function; the function of protecting the medicament, such as, for example, insulating it from the air and/or moisture; the function of filling a pill, capsule or any other form of presentation, such as, for example, dibasic calcium phosphate; a disintegrating function, to facilitate the dissolution of the components and the absorption thereof in the intestine, without excluding other types of excipients not mentioned in this paragraph.

The term "pharmacologically acceptable" refers to the fact that the compound is permitted and evaluated, such that it does not cause any damage to the bodies whereto it is administered.

The term "pharmaceutically acceptable" refers to the fact that the compound allows for the activity of the multipotential stem cell, mesenchymal cell or Nestin-positive mesenchymal cell of the present invention.

The term "active substance" refers to an active substance that must allow for the activity of any of the cells of the invention, i.e. it must be compatible with the multipotential stem cell, mesenchymal cell or Nestin-positive mesenchymal cell.
I.e. at least one multipotential stem cell, mesenchymal cell or Nestin-positive mesenchymal cell is formulated in an appropriate pharmaceutical and pharmacological composition (medicament), in a therapeutically effective quantity. The medicament may be formulated jointly with one or more pharmaceutically and pharmacologically acceptable vehicles, adjuvants or excipients, and may also comprise another active substance.

In every case, the form of presentation of the medicament will be adapted to the type of administration used; for this reason, the composition of the present invention may be presented in the form of solutions or any other clinically permitted administration form.

The medicament of the present invention may be presented in a form adapted for oral or parenteral administration.

The form adapted for oral administration refers to a physical state that allows for the oral administration thereof. The form adapted for oral administration is selected from the list that comprises, without being limited thereto, drops, syrup, tisane, elixir, suspension, extemporaneous suspension, drinkable vial, tablet, capsule, granules, cachet, lozenge, pill, troche or lyophilisate.

The form adapted for parenteral administration refers to a physical state that allows for the injectable administration thereof, i.e. preferably in the liquid state. Parenteral administration may be performed by intramuscular, intraarterial, intravenous, intradermal, subcutaneous or intra-osseous route, without being solely limited to these types of parenteral administration routes.

Another possibility is that the medicament be presented in a form adapted for sublingual, nasal, intrathecal, bronchial, lymphatic, transdermal or inhaled administration.

Another preferred embodiment of the present invention relates to the use of at least one multipotential stem cell, mesenchymal cell or Nestin-positive mesenchymal cell, where the mammal is a human being.

Another aspect of the present invention relates to a method for the maintenance of haematopoiesis *in vitro,* which comprises:
a) placing at least one isolated multipotential stem cell in contact with at least one isolated haematopoietic stem cell, and
b) incubating the product obtained in section (a) in an adequate culture medium for the division and/or differentiation of the haematopoietic stem cell.

As shown in the examples section of the present invention, the multipotential stem cell, preferably the mesenchymal stem cell and, more preferably, the Nestin-positive mesenchymal cell share the same niche as the haematopoietic stem cells in the bone marrow. Therefore, the first step of the method for the maintenance of haematopoiesis *in vitro* involves placing said isolated multipotential stem cell in contact with at least one haematopoietic stem cell in any stage of development prior to the differentiation thereof.

The incubation of said cell mixture must be performed in a culture medium that allows for the division and/or differentiation of the haematopoietic stem cell.

In the present invention, "adequate culture medium" is understood to mean any solution that comprises necessary nutrients for the division and/or differentiation, or for the recovery or isolation, of any of the cells derived from the haematopoietic stem cells of the present invention. Said culture is performed under favourable temperature and pH conditions. The culture medium is selected, without being limited thereto, from the list that comprises DMEM (Dulbecco's Modified Eagle's Medium), RPMI 1640, F12, F10, MCDB 131, MEM (Minimum Essential Media) or DMEM/F12. Moreover, the culture medium may be supplemented with other components, such as, for example, without being limited thereto, CO₂, O₂, serum or serum substitute, amino acids, antibiotics, etc. However, any culture medium known in the state of the art may be used to culture the haematopoietic stem cells. Some types of culture media are described in the examples section of the present invention.

The incubation described in section (b) of the method for the maintenance of haematopoiesis *in vitro* must be performed during the desired period of time, provided that the adequate conditions for said maintenance of haematopoiesis are maintained. To this end, periodical renewal of said medium, or the addition of new culture medium, or the recovery of part of the medium wherein the cells derived from any of the haematopoietic stem cells of the present invention are found may be necessary.

A preferred embodiment of the present invention relates to the method for the maintenance of haematopoiesis *in vitro,* where the multipotential stem cell is a mesenchymal cell. Another preferred embodiment of the present invention relates to the method where the mesenchymal stem cell is a Nestin-positive cell. According to another preferred embodiment of the method for the maintenance of haematopoiesis *in vitro,* the stem cell constitutes an isolated cell population.

Another aspect of the present invention relates to a method for determining the maintenance of the haematopoietic capacity of a mammal, which comprises:
a) analysing at least one expression product of the gene that encodes the Nestin protein in the cells of a sample of haematopoietic tissue isolated from said mammal, and
b) assigning the presence of said expression product analysed in section (a) to the maintenance of the haematopoietic capacity of the organ wherefrom said tissue is derived.

The term "expression product", as understood in the present invention, refers to any product resulting from the expression of the nucleotide sequence that encodes the Nestin protein of the mammal. Thus, the resulting expression product is understood to mean, for example, the messenger RNA that is obtained from the transcription of the nucleotide sequence, the processed messenger RNA, the protein resulting from the translation of any of the messenger RNAs or the cDNA sequence (DNA complementary to the messenger RNA sequence).

The analysis of the expression product is performed by means of any technique known in the state of the art.

The presence of the expression product described in a preceding paragraph indicates the production of the Nestin protein in the cells of the tissue isolated from said mammal. The detection of said presence is dependent on the sensitivity of the detection technique used in the analysis.

A preferred embodiment of the present invention relates to a method for determining the maintenance of the haematopoietic capacity of a mammal, which comprises:
a) determining the concentration of at least one expression product of the gene that encodes the Nestin protein in the cells of a sample of haematopoietic tissue isolated from said mammal,
b) calculating the difference between the concentration of the expression product of the gene that encodes the Nestin protein determined in section (a) and the concentration of the expression of said gene in a positive and/or a negative control, and
c) assigning a significant difference with respect to the positive control, and/or the absence of a significant difference with respect to the negative control, calculated in section (b), to the maintenance of the haematopoietic capacity of the tissue wherefrom said sample is derived.

The negative control is, for example, without being limited thereto, a sample of haematopoietic tissue where the cells are not capable of maintaining haematopoiesis, i.e. where said cells are not capable, for example, of maintaining cellularity within certain desired percentages (known by persons skilled in the art) for any type of cell derived from a haematopoietic stem cell. The desired cellularity percentages may be, without being limited thereto, percentages below 20%, i.e. the negative control presents a dysfunction of the maintenance of haematopoiesis.

The positive control is, for example, without being limited thereto, a sample of haematopoietic tissue where the cells are capable of maintaining haematopoiesis, i.e. where said cells are capable, for example, of maintaining cellularity within certain desired percentages (known by persons skilled in the art) for any type of cell derived from a haematopoietic stem cell. The desired cellularity percentages may be, without being limited thereto, percentages greater than 20%, i.e. the positive control presents the capacity to maintain haematopoiesis.

The term "significant difference", as used in the present invention, refers to a difference calculated in section (b) of the method that is greater than a pre-defined standard error multiplied by a pre-defined security. The pre-defined security may have a value, for example, without being limited thereto, of 95% (p < 0.05) or 99% (p < 0.01). On the contrary, the "absence of a significant difference" refers to a difference calculated in section (b) of the method that is equal to or less than a pre-defined standard error multiplied by a pre-defined security.

Another preferred embodiment relates to the method for evaluating the haematopoietic capacity of a mammal, where said mammal is a human being.

Both multipotential Nestin-positive stem cells and haematopoietic stem cells are adult cells, and were obtained without destroying any embryos or affecting the viability thereof. Throughout the description and the claims, the word "comprises" and the variants thereof are not intended to exclude other technical characteristics, additives, components or steps. For persons skilled in the art, other objects, advantages and characteristics of the invention will arise partly from the description and partly from the practise of the invention. The following figures and examples are provided for illustrative purposes, and are not intended to limit the scope of the present invention.

### DESCRIPTION OF THE FIGURES

In order to complement the description that has been made, as well as contribute to a better understanding of the characteristics of the invention, in accordance with some of the embodiments thereof, the following figures are shown below, for illustrative, non-limiting purposes:

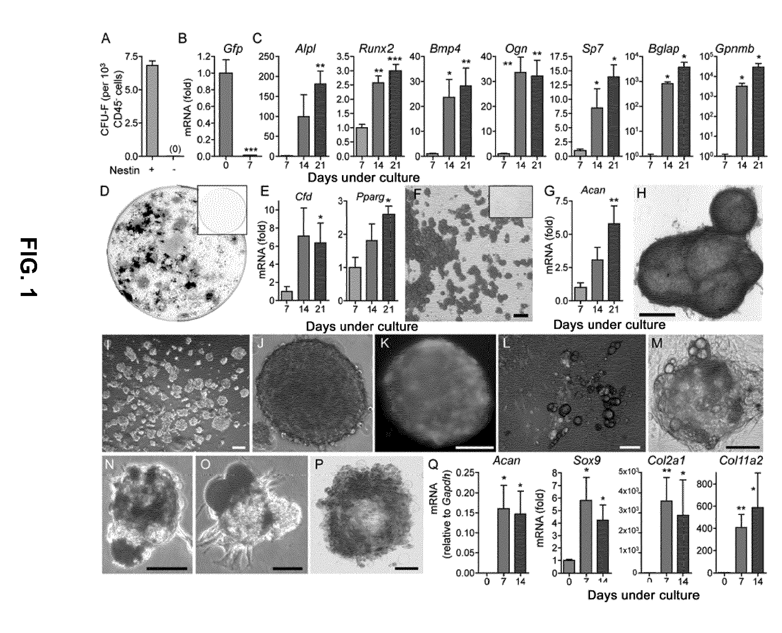

Figure 1.- Shows that Nes:GFP⁺ cells are mesenchymal stem cells.
(A) n = 12.
(B)n=4.
(C)n=4.
(D) Cells isolated as CD45- Nes:GFP⁺ and CD45⁻ Nes:GFP- (box) cultured for 4 weeks with an osteoblast differentiation medium.
(E) Progressive differentiation into adipocytes from CFU-Fs obtained from CD45⁻ Nes:GFP⁺ cells cultured for 3 weeks with adipocyte differentiation medium (n = 4).
(F) CD45⁻ Nes:GFP- cells did not generate any colonies (box).
(G) *Acan*: expression of aggrecan during the 3 weeks of culture with chondrocyte differentiation medium.
(H) Accumulation of Alcian blue⁺ mucopolysaccharides in cell pellets from CD45⁻ Nes:GFP⁺ cells cultured for 3 weeks with chondrocyte differentiation medium (n = 4).
(I) Clonal spheres: The CD45⁻ Nes:GFP⁺ cells following 7 days of low-density culture; bright field.
(J-K) Expression of GFP in a sphere after 10 days of culture; (J) bright field; (K) fluorescence.
(L) Differentiation of CD45⁻ Nes:GFP⁺ cells into mesenchymal lineages.
(M) Multi- and unilocular refringent adipocytes, as well as cells migrating from the outer layer of the sphere, adhered to the surface of the culture plate.
(N-O) Clonal mesenspheres (n = 51) differentiated into Col2.3-LacZ⁺ OBs and Oil red O⁺adipocytes.
(P) Representative example of clonal mesensphere that accumulated Alcian blue⁺ mucopolysaccharides after 3 weeks under culture with chondrocyte differentiation medium (n = 4).
(Q) Progressive differentiation of the clonal mesenspheres cultured for 3 weeks in chondrocyte differentiation medium (n = 5-11), manifested by the increased expression of (*sex determining region Y*)-box 9 (Sox9), aggrecan (*Acan*) and type IIα1 (*Co*/*2α1*) and type XIα2 (*Col11α2*) collagens.
Scale, 100 µm (F, I, K, N, P); 500 µm (H); 50 µm (L-M, O). * p < 0.05; ** p < 0.01; *** p < 0.001; two-tailed unpaired t-test; the error bars indicate the standard error.

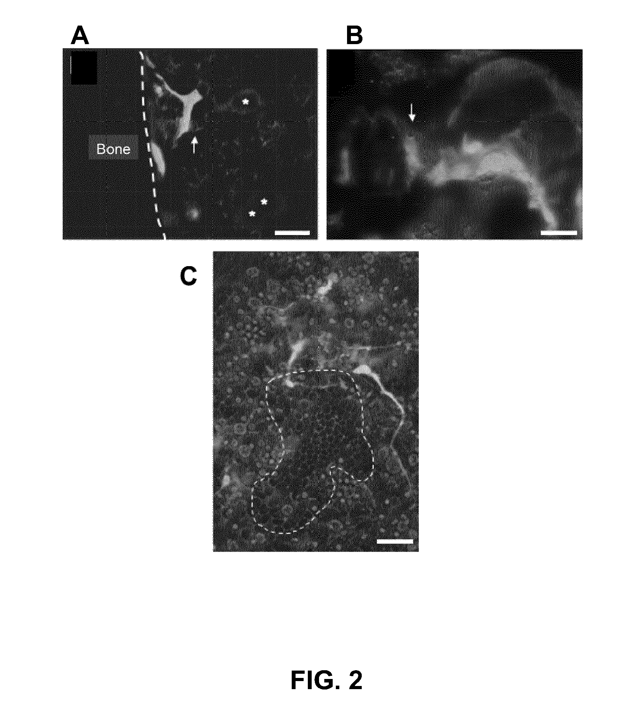

Figure 2.- Shows that the Nes:GFP⁺ cells are physically associated with the HSCs.
(A, B) Immunohistochemistry against CD150 (arrows), CD48 and haemopoietic lineage markers in bone marrow sections of Nes-Gfp mice. Representative images of CD150⁺ CD48- Lin- HSCs located adjacent to the Nes:GFP⁺ cells of the endostium (A) and the sinuses (B); (A) The CD150⁺ CD48 / Lin⁺ megakaryocytes, indicated with asterisks, were easily identified thanks to their large size and homogeneous CD150 staining. Deconvolutions of the Z projections.
(C) The scant Nes:GFP⁺ cells detectable in primary myeloid cultures were frequently associated with cobblestone-forming areas, rich in haematopoietic progenitors. Combined fluorescence and phase contrast image.
Scale, 50 µm.

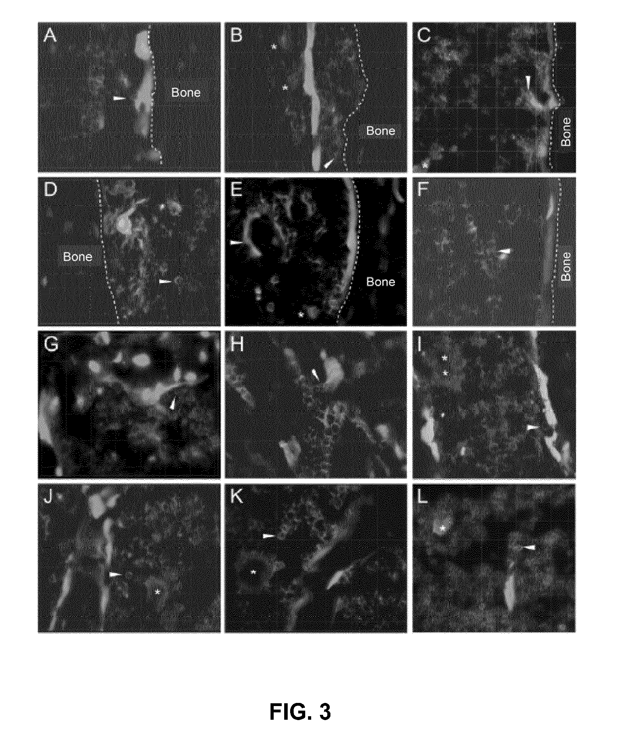

Figure 3.- Shows that the HSC cells are located close to the Nes:GFP+ cells in the BM. Immunohistochemistry against CD15; Ter119, Gr-1, CD3e, B220 and Mac-1 (Lin), and CD48 in bone marrow sections of *Nes-Gfp* transgenic mice. The tips of the arrows indicate examples of CD150+ CD48-Lin- HSCs. The HSCs were frequently located adjacent to (A, C) or close to (B, D) the Nes:GFP+ cells of the endostium, and most of them in direct contact with (E, G-I) or close to (J-L) the Nes:GFP+ cells that surrounded the sinuses. The asterisks indicate CD150+ CD48+ megakaryocytes, easily identifiable by their large size and homogeneous cytoplasmic distribution of CD150. Grid, 50 µm.

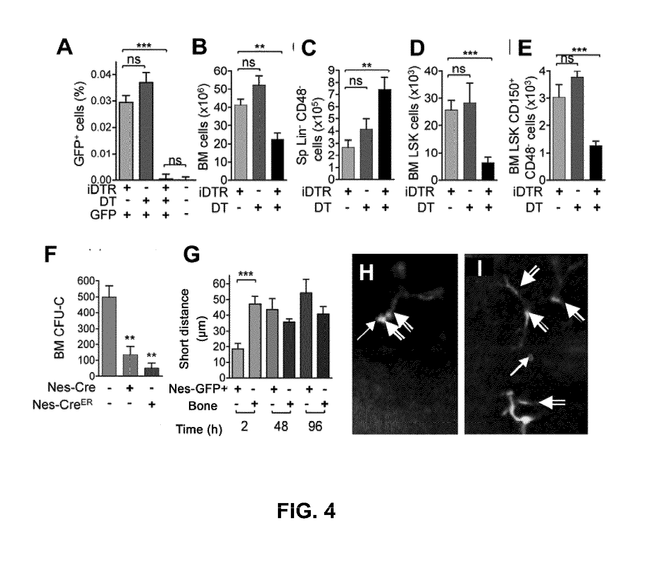

Figure 4.- Shows that the selective elimination of cells that express nestin sharply reduces the content and nesting of HSC cells in the bone marrow.
(A) n = 5-10.
(B) n = 5-7.
(C) n = 3-8.
(D-E) n = 4-5.
(F) n = 4-8.
(G) Shortest mean distances between the Nes:GFP⁺ cells and the HSCs nested in the bone marrow, or between these and the bone wall, 2 h (n = 16), 48 h (n = 30) and 96 h (n = 14) later.
(H-I) Representative examples of HSCs stained with DyD (thin arrow) nested in less than 2 h close to GFP⁺ cells (thick arrow) in the bone marrow of *Nes-Gfp* mice. The bone matrix was visualised by means of the second harmonic signal generated by collagen when it is illuminated by femtosecond pulses with a titanium:sapphire laser.
* p < 0.05, ** p < 0.01, *** p < 0.001; two-tailed unpaired t-test; the error bars indicate standard error.

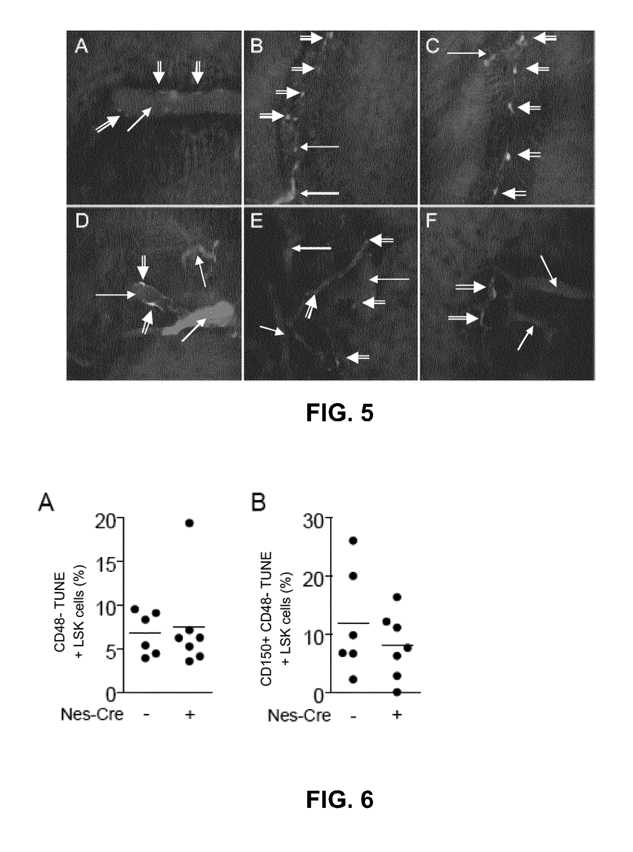

Figure 5.- Shows that the Nes:GFP+ cells in the cranial bone marrow are perivascular. Intravital microscopy of the calvaria of Nes-Gfp transgenic mice injected with Qdot by i.v. route (thin arrow).
(A-C) Nes:GFP+ cells (thick arrow) surrounding the vessels of the coronary suture.
(D-F) Nes:GFP+ cells in the endostium.

Figure 6.- Shows that selective cellular depletion in Nes-Cre/iDTR mice does not alter the cell cycle of the haemopoietic progenitors. Frequency of CD48-, Lin-Sca-1+ c-kit+ (LSK) (A) and CD150+ CD48- LSK (B) cells in the bone marrow of Nes-Cre/iDTR double transgenic mice or iDTR control animals 24 h after the injection of diphtheria toxin (DT). The bone marrow samples, stained as described in the section on Materials and Methods, were fixed and permeabilised, and TUNEL was detected using the Mebstain Apoptosis Kit Direct (MBL) and FACS. Each point corresponds to a mouse. ** p < 0.01, two-tailed unpaired t-test.

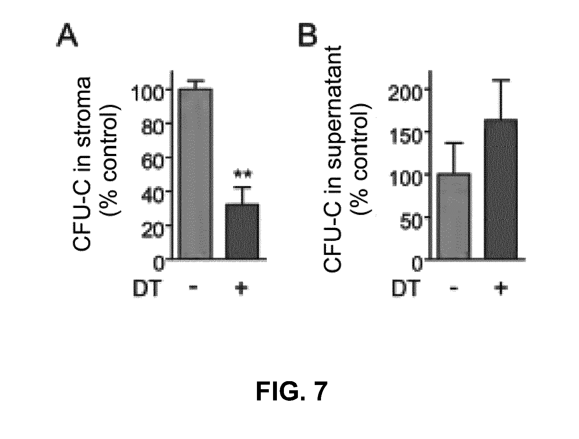

Figure 7.- Shows that selective cellular depletion in the long-term cultures from the bone marrow of Nes-Cre/iDTR mice reduces the number of haemopoietic progenitors. The number of colony-forming units in culture (CFU-C) was determined in the adherent fraction (A) and the supernatants (B) of myeloid bone marrow cultures (Dexter type) from Nes-Cre / iDTR double transgenic mice. The diphtheria toxin (DT, 100 ng/ml) was added to the medium when the cultures were seeded and at the weekly changes of half of the culture medium. The data were normalised on the basis of similar bone marrow cultures from iDTR control animals in order to discard the non-specific toxicity of the DT *in vitro* (n = 3 mice). ** p < 0.05; two-tailed unpaired t-test. The error bars indicate standard error.

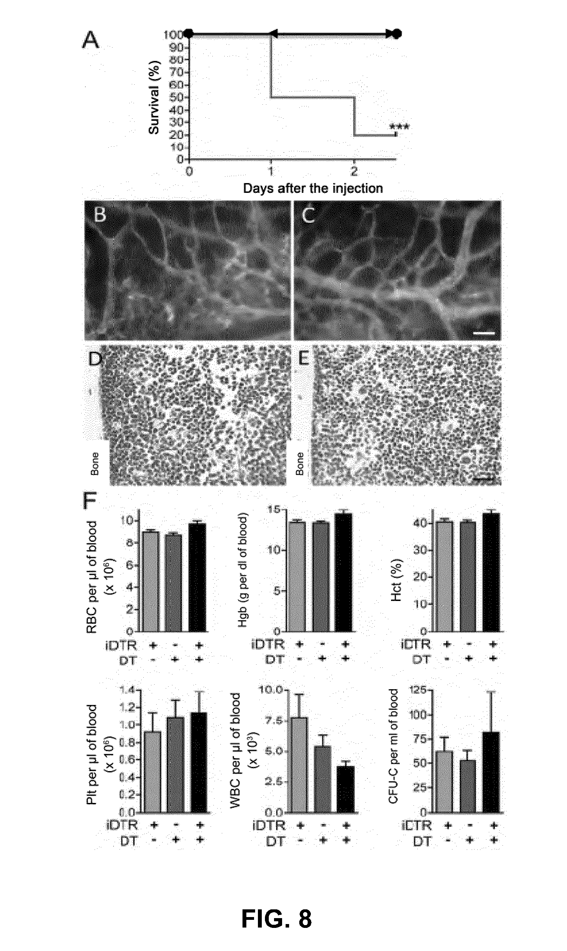

Figure 8.- Shows that selective cellular depletion in *Nes-CreliDTR* mice reduces the survival but does not affect the vascular permeability or the histology of the bone marrow or the haemogram.
(A) Reduced survival of the *Nes-CreliDTR* mice following selective cell ablation. Survival percentage of the *Nes-Cre* / *iDTR* and *Nes-Cre* / *iDTR* / *ROSA-Gfp* mice triple-injected with diphtheria toxin (DT, 4 µg/kg, i.p.; line without elements at the ends) or PBS (line with arrow tips at both ends) (n = 10), as compared to control animals treated with DT (line with black circles at both ends, n = 5). *** p < 0.001 (Log-rank test).
(B-C) FITC-Dextran dye, prepared and injected by i.v. route in the *Nes-Cre* / *iDTR* animals treated with DT in the vasculature of the cranial bone marrow (C), or in control animals (B).
(D-E) Bone marrow histology of the *Nes-Cre* / *iDTR* animals treated with DT (E) and histology of the control animals (D). Representative examples of n = 4 mice. Scale, 100 µm (B-C); 50 µm (D-E).
(F) Quantity of erythrocytes (RBC), haemoglobin (Hgb), haematocrit (Hct), platelets (PIt), leukocytes (WBC) and haemopoietic progenitors (CFU-C) in the blood of *Nes-Cre* / *iDTR* and *Nes-Cre* / *iDTR* / *ROSA-Gfp* mice 24-48 h after the treatment with DT, as compared to the levels observed in transgenic animals injected with PBS or in control mice treated with DT (n = 4-7). Two-tailed unpaired t-test. The error bars indicate standard error.

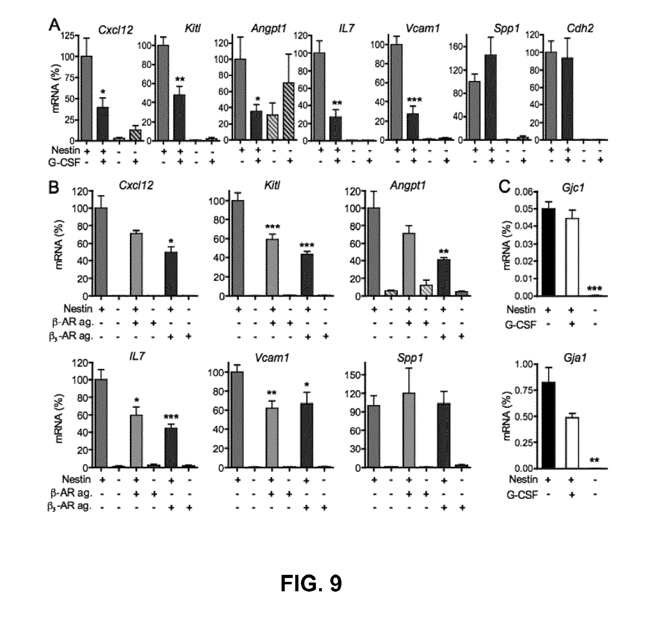

Figure 9.- Shows the expression of critical genes for the maintenance of HSCs. Quantitative RT-PCR from RNA samples obtained from the CD45⁻ GFP⁺ and CD45⁻ GFP- cells isolated from the bone marrow of *Nes-Gfp* transgenic mice, in order to determine the expression of *Cxc*/*12,* stem cell factor/kit ligand *(KitI),* interleukin-7 *(IL7),* vascular cell adhesion molecule-1 *(Vcam1),* osteopontin *(Spp1)* and N-cadherin *(Cdh2), Gapdh,* angiopoietin-1 *(Angpt1),* in the Nes:GFP+ cells and the rest of the CD45- bone marrow population.
(A) n = 6.
(B) The animals were injected with saline, a non-selective b-adrenergic agonist (isoproterenol, b-AR) or a selective β₃ adrenergic receptor agonist (BRL37344; (β₃-AR) (2 mg/kg, i.p.) and were sacrificed 2 h later. (n = 6).
(C) Expression of connexins 45 (Gjc1) and 43 (Gja1). (n = 3).
* p < 0.05, ** p < 0.01, *** p < 0.001; two-tailed unpaired t-test; the error bars indicate standard error.

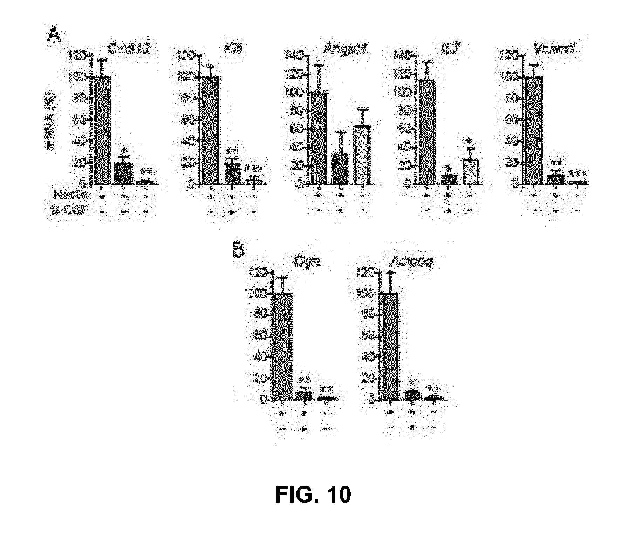

Figure 10.- Shows the results of quantitative RT-PCR using a different housekeeping gene.
(A-B) Quantitative PCR was performed from RNA samples obtained from the CD45- GFP+ and CD45- GFP- cells isolated from the bone marrow of *Nes-Gfp* transgenic mice. Very reproducible results were obtained using *β-actin* instead of *Gapdh* as the housekeeping gene. Expression of critical genes for the maintenance of HSCs (A) or for mesenchymal differentiation (B). (n = 3-7). * p < 0.05, ** p < 0.01, *** p < 0.001; two-tailed unpaired t-test. The error bars indicate standard error.

Figure 11.- Shows a representative human mesensphere.

Figure 12.- Shows the osteoblast differentiation of human mesenspheres. We may observe the alkaline phosphatase and Von Kossa stainings of the CFU-F derived from each human primary mesensphere cultured under osteoblast differentiation conditions.

### EXAMPLES

Below we will illustrate the invention by means of illustrative, non-limiting assays performed by the inventors, which describe the use of the multipotential cells of the present invention for the maintenance of haematopoiesis.

### EXAMPLE 1. Determination of the fact that Nes:GFP+ cells (Nestin-positive) are mesenchymal stem cells (MSCs)

In the first place, the hypothesis was raised that Nes:GFP+ cells were genuine MSCs. In fact, the entire fibroblast colony-forming units (CFU-F) and clonogenic capacity of the bone marrow resided in the Nes:GFP⁺ fraction (Fig. 1A). The culture of all the remaining CD45⁻ Nes:GFP- bone marrow cells at equal or greater density did not generate any CFU-F. After seeding the CD45⁻ Nes:GFP⁺ cells under different culture conditions, including "MSC" medium, the cells quickly lost the expression of GFP (Fig. 1B) and differentiated into mesenchymal progenitors, as was determined using the CFU-F assay. In order to more exhaustively analyse their differentiation potential, the CD45⁻ Nes:GFP⁺ and CD45⁻ Nes:GFP- cells isolated by FACS were seeded under conditions that favour differentiation into osteoblast, adipocyte and chondrocyte lineages. The CD45⁻ Nes:GFP⁺ fraction showed a robust capacity to differentiate into mesenchymal lineages, whereas the CD45⁻ Nes:GFP- fraction did not generate any offspring. Different genes necessary for differentiation into OBs (Fig. 1 C), adipocytes (Fig. 1 E) and chondrocytes (Fig. 1G) were significantly upwardly regulated in CD45⁻ Nes:GFP⁺ cells for 3 weeks of culture with a differentiation medium. The mature, mineralising, adipocytic or chondrocytic phenotype was confirmed after one month under culture (Fig. 1D, F, H), thereby demonstrating their capacity to differentiate into multiple lineages.

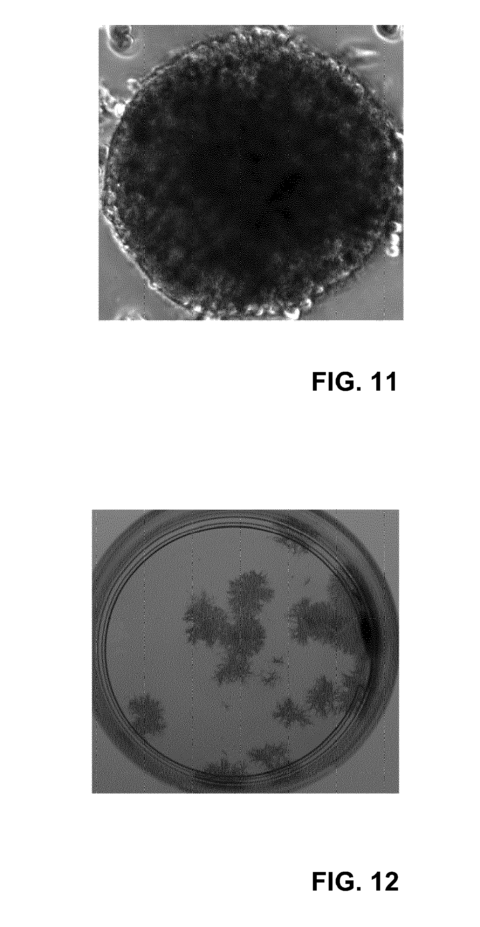

Since neural stem cells, which express nestin, may self-renew when they are cultured as floating spheres (Stemple & Anderson, 1992. Cell. Dec 11; 71 (6): 973-85), we adapted culture conditions of neural crest stem cells (Pardal et al., 2007. Cell, 131: 364-377) and pericytes (Crisan et al., 2008. Curr Protoc Stem Cell Biol, Chapter 2, Unit 2B 2 1-2B 2 13) in order to expand the Nes:GFP+ cells in low-attachment culture plates. The CD45⁻ Nes:GFP⁺ cells, but not the CD45⁻ Nes:GFP- cells (isolated by FACS), formed clonal spheres when they were seeded at low density (Fig. 11). These mesenchymal spheres, herein called "mesenspheres", had a mean diameter of 85 ± 6 µm and continued to express GFP after 7-10 days under culture (Fig. 1J-K). The sphere formation efficiency (6.5 ± 0.7%) was similar to that of neural crest stem cells (Molofsky et al., 2003. Nature, 425: 962-967). The frequency of the spheres was remarkably similar (6.9 ± 0.7 %) when the CD45⁻ Nes:GFP⁺ cells were seeded in 96-well plates (1 cell / well). The self-renewal capacity of CD45⁻ Nes:GFP⁺ cells was studied by dissociating the mesenspheres and seeding them under the same conditions. A mean of 4.4 ± 0.8 secondary mesenspheres were obtained from each primary sphere (n = 19), a frequency lower than that described from neural stem cells (Molofsky et al., 2003. Nature, 425: 962-967), possibly due to the dense extracellular matrix produced by CD45⁻ Nes:GFP⁺ cells, which hinders an effective dissociation of the spheres without compromising the cell viability. After two weeks under culture, the clonal mesenspheres, as well as the multiclonal cultures from CD45⁻ Nes:GFP⁺ cells (Fig. 1L), continued to proliferate but showed a progressive loss of the expression of GFP and began to differentiate into mesenchymal lineages (Fig. 1M). In order to more exhaustively study the mesenchymal differentiation capacity of CD45⁻ Nes:GFP⁺ cells, we crossed *Nes-Gfp* transgenic mice with a line that expresses Cre recombinase under the 2.3-kb proximal fragment of the collagen α1(I) promoter (*Col2*.*3*-*Cre*), selectively expressed in OBs (Dacquin et al., 2002. Dev Dyn, 224: 245-251), and with the *ROSA26* / *IoxP-stop-IoxP-LacZ* (*R26R*) reporter line. After two weeks under culture, the clonal mesenspheres obtained from triple transgenic animals showed spontaneous differentiation into adipocytes, the identity whereof was confirmed by means of staining with Oil Red O, and into Obs, unequivocally identified by the expression of β-galactosidase directed by the Co/2.3 promoter (∼ 53%; 27/51; Fig. 1N-O). When the mesenspheres that had already shown signs of adipocyte differentiation were seeded under conditions that favour differentiation into chondrocytes, they accumulated mucopolysaccharides characteristic of the cartilage (stained with "Alcian Blue"; Fig. 1P) and increased the expression of the genes necessary for chondrocyte differentiation (Fig. 1Q). These data clearly show that the Nes:GFP⁺ mesenspheres are self-renewable and multipotent *in vitro.*

### Conclusions according to Fig. 1:

According to Fig. 1A, the entire capacity of the bone marrow to generate fibroblast colony-forming units (CFU-F) or mesenchymal activity is contained in the Nes:GFP⁺ population (n = 12). In Fig 1B, the expression analysis by Q-PCR shows a rapid reduction in the expression of *Gfp* one week after seeding the cells with CFU-F formation medium.

Fig. 1C shows different determination markers towards the osteoblast lineage *(alkaline phosphatase, Alpl; Runx2)* and genes associated with the differentiation into OBs (bone morphogenetic protein-4, *Bmp4;* osteoglycin, *Ogn*; osterix, *Sp7*; osteocalcin, *Bglap;* osteoactivin, *Gpnmb).* Said markers showed a progressive increase in expression in CFU-Fs derived from the isolated CD45⁻ Nes:GFP⁺ cells cultured for 4 weeks in osteoblast differentiation medium. In Fig. 1D, we may observe the confirmation of the presence of osteoprogenitor cells only in the CD45⁻ Nes:GFP⁺ population by means of the detection of the activity of alkaline phosphatase and the calcium deposits. The CD45⁻ Nes:GFP- cells did not generate any colonies. In Fig. 1E, we may observe the progressive differentiation into adipocytes from CFU-Fs obtained from CD45⁻ Nes:GFP⁺ cells cultured for 3 weeks with adipocyte differentiation medium, made evident by an increase in the expression of adipsin *(Cfd)* and peroxisome proliferator-activated receptor gamma 2 (*Pparg*). Fig. 1F shows that the mature differentiated phenotype of the adipocytes derived from the isolated CD45⁻ Nes:GFP⁺ cells was confirmed in the 4-week-long cultures by means of Oil Red O staining; on the contrary, the CD45⁻ Nes:GFP- cells did not generate any colonies (box). Fig. 1G shows that the progressive differentiation of the CFU-Fs obtained from the isolated CD45⁻ Nes:GFP⁺ cells into chondrocytes was manifested in an increase in the expression of aggrecan during the 3 weeks of culture with chondrocyte differentiation medium (*Acan*). In Fig. 1H, we may observe the accumulation of Alcian blue⁺ mucopolysaccharides in cell pellets derived from CD45⁻ Nes:GFP⁺ cells cultured for 3 weeks with chondrocyte differentiation medium. As shown in Fig. 1l, the CD45⁻ Nes:GFP⁺ cells, but not the rest of the CD45⁻ bone marrow population, form clonal spheres after 7 days under low-density culture. Fig. 1L shows that the CD45⁻ Nes:GFP⁺ cells isolated and cultured together in polystyrene plates quickly lost the expression of GFP and differentiated into mesenchymal lineages; the GFP-adipocytes were clearly shown by the refringent lipid deposits under bright field. In Fig. 1M, we may observe multi- and unilocular refringent adipocytes, as well as cells migrating from the outer layer of the sphere, adhered to the surface of the culture plate. Fig. 1N-O shows that, after 3 weeks under culture, 53% of the clonal mesenspheres showed signs of multi-lineage differentiation into CoI2.3-LacZ⁺ OBs and Oil red O⁺ adipocytes. In Fig. 1Q, we may observe the progressive differentiation of the clonal mesenspheres cultured for 3 weeks in chondrocyte differentiation medium, manifested in the increased expression of *(sex determining region* Y)-box 9 (*Sox9*), aggrecan (*Acan*) and collagens type Ilα1 *(CoI2α1)* and type XIα2 *(CoI11α2).*

### EXAMPLE 2. Demonstration that Nes:GFP+ cells are different from other stem cells

In order to elucidate the origin and identity of the Nes:GFP⁺ MSCs, we performed comparative analyses of the complete genome of CD45⁻ Nes:GFP⁺ bone marrow cells and that of different stem cells, obtained from the "Gene Expression Omnibus" (http://www.ncbi.nim.nih.gov/geo/) and "Stem-base" (http://www.stembase.ca/?path=/) databases. These analyses comprised human and murine MSCs, mouse embryonic and neural stem cells, multipotent murine cells derived from embryonic carcinomas, mouse satellite cells and human haematopoietic stem cells (HSCs) (Table 1). In order to compare the data from the human and murine genomes, the mouse genes that are homologous to human genes were identified using HomoloGene (http://www.ncbi.nlm.nih.gov/homologene). As a result, the expression of 9,000 paired genes annotated in databases was finally used for the study. The impartial hierarchical clustering and principal component analyses revealed that CD45⁻ Nes:GFP⁺ bone marrow cells were very different from adult neural stem cells. Although CD45⁻ Nes:GFP⁺ cells are different from all the stem cells included in the analysis, their expression pattern was closer to that of human CD45⁻ CD146⁺ osteoprogenitor cells (Sacchetti et al., 2007. Cell, 131: 324-336). Table 1. List of experiments with DNA chips and their accession numbers m, mouse; BM, bone marrow; NSC, neural stem cell; h, human; FB, foetal blood; PB, peripheral blood; EB, embryoid bodies; MSC, mesenchymal stem cell; ESC, embryonic stem cell.

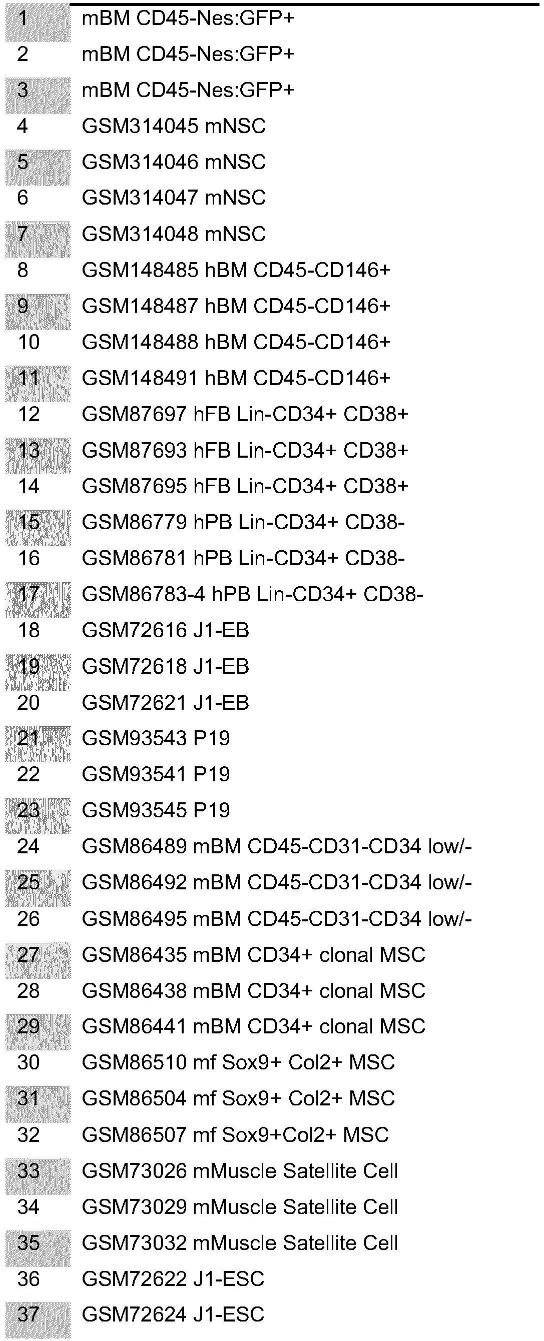

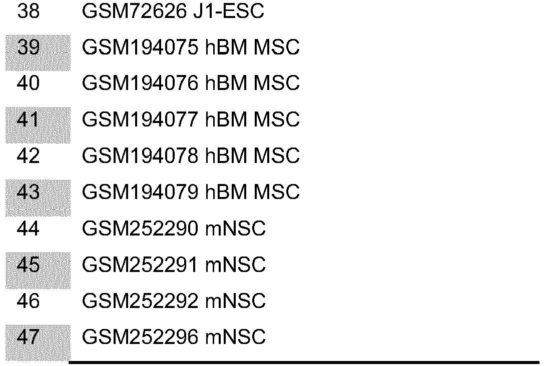

Gene ontology analyses were performed on the transcripts that were significantly upwardly or downwardly regulated in the CD45⁻ Nes:GFP⁺ bone marrow cells, as compared to all the other stem cells studied. These analyses revealed that most of the upwardly regulated genes were involved in metabolic and biosynthetic processes, whereas most of the downwardly regulated genes were involved in mitosis and cell division. These results indicate that CD45⁻ Nes:GFP⁺ bone marrow cells are relatively quiescent but metabolically very active.

### EXAMPLE 3. Demonstration that Nes:GFP+ cells form a single perivascular niche

HSCs may be identified and isolated with great purity using a combination of SLAM markers (Kiel et al., 2005. Cell, 121: 1109-1121). In order to evaluate the spatial relationship between Nes:GFP+ cells and HSCs, immunohistochemistry was performed on bone marrow cryocuts from *Nes-Gfp* transgenic mice using haemopoietic lineage markers (anti-Ter119, Gr-1, CD3e, B220 and Mac-1), CD48 and CD150 (Figs. 2A-B and Fig. 3). The CD150⁺ CD48- Lin- HSC cells represented a very small subpopulation (∼ 0.005%) of the nucleated bone marrow cells. Despite the low frequency of both HSCs and Nes:GFP+ cells, the great majority (88%; 37/42) of the CD150⁺ CD48- Lin-cells were located less than 5 cell diameters from the Nes:GFP+ cells, and most of them (60%; 25/42) were located directly adjacent to the Nes:GFP+ cells distributed in the sinuses (62%) or the endostium (26%; Fig. 2A, B. Fig. 3). The statistical inference from the Poisson distribution expected for the Nes:GFP+ cells in the sampling area demonstrates that the co-location of the CD150⁺ CD48⁻ Lin⁻ and Nes:GFP⁺ cells observed is highly significant (p < 10⁻¹⁶) . Total bone marrow was cultured under conditions that promote myeloid growth (Dexter type), in order to determine whether Nes:GFP+ cells contribute to the stromal layer. Despite the low number of Nes:GFP+ cells detectable in these cultures, these cells were frequently located in association with cobblestone-forming areas, rich in haemopoietic progenitors (Fig. 2C). Therefore, these results suggest a close physical association between Nes:GFP+ cells and HSCs in the BM.

### EXAMPLE 4. Demonstration that nestin+ cells are necessary for the nesting of HSCs in the BM.

The high expression of *CxcI12, Vcam1, KitI, II7* and *Angpt1* in CD45⁻ Nes:GFP⁺ cells and the marked reduction thereof during the mobilisation induced by the granulocyte colony-stimulating factor (G-CSF) or following β-adrenergic stimulation suggested that nestin⁺ cells could regulate the attraction of HSCs and their traffic in the bone marrow. In order to determine whether nestin⁺ cells played a significant role in the nesting of HSCs in the bone marrow, 5 x 10⁶ nucleated bone marrow cells from wild-type congenic animals were transplanted into lethally irradiated *Nes-Cre* / *iDTR* mice, *Nes-Cre^{ERT2}* / *iDTR* mice and *iDTR* controls, following a previously described protocol (Katayama et al., 2006. Cell, 124: 407-421). The nesting of CFU-Cs in the bone marrow was reduced by 73% in the *Nes-Cre* / *iDTR* mice and by 90% in the *Nes-Cre^{ERT2}* / *iDTR* animals (Fig. 4N), which indicates that nestin⁺ cells are in fact necessary for the nesting of haemopoietic progenitors in the BM.

In order to determine whether nestin⁺ bone marrow cells directly regulated the nesting of HSCs, the migration of previously isolated HSCs with a high degree of purification, and subsequently labelled and trasplanted into lethally irradiated *Nes-Gfp* transgenic mice, was visualised by means of intravital microscopy. Since these *in vivo* image studies can only be performed in the cranial bone marrow, but not in the long bones (Lo Celso et al., 2009. Nature, 457: 92-96), we first validated the calvaria model by comparing the frequency, distribution and physiological properties (capacity to form CFU-C and mesenspheres) of the CD45⁻ Nes:GFP⁺ cells isolated from the bone marrow of the cranium and the long bones. The Nes:GFP⁺ calvarial cells were also perivascular, as verified by intravital microscopy of *Nes-Gfp* transgenic mice injected with Qdot by i.v. route in order to stain the vasculature (Fig. 5). The mesensphere formation frequency of the CD45⁻ Nes:GFP⁺ cells obtained from the cranial bone marrow was identical to that found in the bone marrow of long bones, and also exclusive within the CD45⁻ population. The CD45⁻ Nes:GFP⁺ cells also contained all the CFU-F activity of the cranial bone marrow. The frequency and morphology of the Nes:GFP⁺ cranial bone marrow cells were not affected by the lethal irradiation, in agreement with the characteristic radio-resistance of MSCs. The CD150⁺ CD48⁻ LSK cells were isolated by flow cytometry, stained with a fluorescent lipophilic dye and injected by i.v. route in lethally irradiated adult *Nes-Gfp* transgenic mice. The nesting of HSCs in the bone marrow was directly visualised in the cranial bone marrow 2, 48 and 96 h later, by a combination of confocal and two-photon microscopy (Lo Celso et al., 2009. Nature, 457: 92-96). The entire cranial bone marrow was scanned and Z projections were obtained, wherefrom the shortest distance between the HSCs that had nested in the bone marrow and the Nes:GFP⁺ cells and the bone surface was calculated (Fig. 4O). The analyses performed clearly showed that the HSCs quickly nested close to the Nes:GFP+ bone marrow cells (Fig. 40-Q), which indicates that nestin⁺ cells direct the migration of HSCs in the bone marrow.

### EXAMPLE 5. Demonstration that Nestin-positive cells are necessary for the maintenance of HSCs in the bone marrow

In order to determine whether nestin⁺ cells are in fact necessary for the maintenance of HSCs in the bone marrow, we attempted to eliminate them by crossing *Nes-Cre* mice with a line that Cre-inducibly expresses the diphtheria toxin (DT) receptor (Buch et al., 2005. Nat Methods, 2: 419-426) *(iDTR)* and with the *ROSA26* / *IoxP-stop-IoxP-GFP* (ROSA-GFP) reporter line (Mao et al., 2001. Blood, 97: 324-326). A single injection of DT (4 µg/kg, i.p.) completely eliminated the GFP⁺ bone marrow cells in 24-48 h (Fig. 4A), and caused a reduction of ∼ 45% in the total bone marrow cellularity (Fig. 4B), associated with a ∼ 40% increase in the number of cells stained with propidium iodide, probably due to the abrupt reduction in cytokines and growth factors that are critical for different haemopoietic progenitors (such as IL-7, the c-kit ligand and M-CSF) and which are produced in large quantities by nestin⁺ bone marrow cells. A 2.3-fold increase in the number of Lin- CD48- haemopoietic progenitors was also detected in the spleen of *Nes-Cre* / *iDTR* double transgenic mice, as compared to single transgenic controls, 24-48 h after the treatment with DT (Fig. 4C), which suggests an extramedullary migration of the haemopoietic progenitors.

It is worth noting that HSCs turned out to be extremely sensitive to selective cellular depletion in *Nes-Cre* / *iDTR* double transgenic mice, as manifested in a ∼ 75% reduction in the number of bone marrow LSK cells (Fig. 4D) and a ∼ 58% reduction in the number of CD150⁺ CD48⁻ LSK cells (Fig. 4E). We studied whether this greater reduction in HSCs was caused by apoptosis, but no differences were detected between the two groups of animals in regards to the frequency of CD48⁻ TUNEL⁺ LSK or CD150⁺ CD48⁻ TUNEL⁺ LSK cells (Fig. 6). The reduction in haemopoietic progenitors in the bone marrow was directly caused by selective cellular depletion in the bone marrow, since treatment of the primary bone marrow cultures of *Nes-Cre* / *iDTR* double transgenic mice with DT reduced the number of haemopoietic progenitors by ∼ 70%, as compared to single transgenic controls (Fig. 7). Treatment *of Nes-Cre* / *iDTR* double transgenic mice and *Nes-Cre* / *ROSA-Gfp* / *iDTR* triple transgenic mice with DT was lethal in 50% of the animals after 24 h and in 80% of them 48 h later (n = 10), whereas it did not affect the viability of *Nes-Cre* / *ROSA-Gfp* double transgenic mice and single transgenic control mice (Fig. 8A). Despite the reduction in total bone marrow cellularity, the vascular permeability, the histology and the haemogram were normal (Fig. 8B-F), which suggests that the mortality was not caused by haematopoietic failure, but by the depletion of nestin⁺ cells or the offspring thereof in vital organs, such as the heart and/or the brain. In Fig. 8B-C, we may observe that the FITC-Dextran dye, prepared and injected by i.v. route, as previously described (von Andrian, 1996), in the Nes-*Cre* / *iDTR* animals treated with DT, remained in the cranial bone marrow vasculature (Fig. 8C), as was also the case in the control animals (Fig. 8B), which indicates the absence of vascular damage. Fig. 8D-E shows that the bone marrow histology of the *Nes-Cre* / *iDTR* animals treated with DT (8E) is similar to that of the control animals (8D). Fig. 8F shows that selective cellular depletion in *Nes-Cre* / *iDTR* mice did not affect the haemogram or the number of circulating progenitors. The quantity of erythrocytes (RBC), haemoglobin (Hgb), haematocrit (Hct), platelets (Plt), leukocytes (WBC) and haemopoietic progenitors (CFU-C) in the blood did not change significantly in the *Nes-Cre* / *iDTR* and *Nes-Cre* / *iDTR* / *ROSA-Gfp* mice 24-48 h after the treatment with DT, as compared to the levels observed in transgenic animals injected with PBS or in control mice treated with DT.

### Conclusions based on Fig. 4

In Fig. 4A-E, we may observe that, following selective cell ablation in *Nes-Cre* / *iDTR* double transgenic mice, the haemopoietic progenitors move from the bone marrow to extramedullary sites. Fig. 4A shows that a single injection of diphtheria toxin (DT, 4 µg/kg, i.p.) in *Nes-Cre* / *ROSA-Gfp* / *iDTR* triple transgenic mice completely eliminated the GFP⁺ bone marrow population after 24-48 h, as revealed by the absence of GFP⁺ cells, and compared to the normal levels in triple transgenic control mice injected with PBS or in *Nes-Cre* / *ROSA-Gfp* double transgenic mice injected with DT. In Fig. 4B, we may observe the reduced cellularity in the bone marrow of the femurs and tibias of *Nes-Cre* / *iDTR* and *Nes-Cre* / *iDTR* / *ROSA-Gfp* mice 24-48 h after the treatment with DT, which did not affect the animals that did not have the *iDTR* transgene, as a control. Fig. 4C shows the detection of a 2.8-fold increase in the number of Lin-CD48- cells detected in the spleen of *Nestin-Cre* / *iDTR* mice 24-48h after the injection with DT, as compared to double transgenic control animals injected with PBS or single transgenic animals treated with DT. In Fig. 4D-E, we may observe a reduction in the number of bone marrow HSC cells in *Nes-Cre* / *iDTR* mice after selective cell ablation. 24-48 h later, treatment of the *Nes-Cre* / *iDTR* and *Nes-Cre* / *ROSA-Gfp* / *iDTR* mice with DT caused a ∼ 75% reduction (D) in the number of Lin- Sca-1⁺ c-kit⁺ (LSK) and a ∼ 58% reduction (E) in the CD150⁺ CD48⁻ bone marrow LSK cells. Figs. 4F-I show that nestin⁺ bone marrow cells are necessary for the nesting of HSCs and haemopoietic progenitors. For example, in Fig. 4F we may observe that the depletion of nestin⁺ cells reduces the nesting of haematopoietic progenitors in the bone marrow. The *Nes-Cre* / *iDTR, Nes-Cre^{ERT2}* / *iDTR* mice and the *iDTR* controls were injected with DT (4 µg/kg, i.p.) and lethally irradiated (1.2 Gy, one dose) 16-20 h later. Five million nucleated cells obtained from the bone marrow of congenic animals were injected by i.v. route in a volume of 200 µl of PBS. The bone marrow was seeded 3 h later in the medium designed for the growth of colony-forming units in culture (CFU-C). The number of CFU-Cs nested for each femur was corrected to represent the total bone marrow by multiplying by 16.9, since a femur contains approximately 5.9% of the total mouse bone marrow, as previously described (Katayama et al., 2006. Cell, 124: 407-421). In each experiment, one or two irradiated animals (of the same age, genotype and gender) did not receive a transplant, in order to correct the data in the event that residual progenitors from the recipient were found. The selective elimination of nestin⁺ cells in the *Nes-Cre* / *iDTR* and *Nes-Cre^{ERT2}* / *iDTR* mice significantly compromised the nesting of haemopoietic progenitors in the bone marrow. On the other hand, Fig. 4G-1 shows that HSCs rapidly nest close to the GFP⁺ bone marrow cells of *Nes-Gfp* transgenic mice. The bone marrow cells of congenic mice were stained with biotinylated antibodies against haemopoietic lineage markers (detected with streptavidin conjugated with Pacific Orange), a-c-kit conjugated with APC, a-Sca-1 conjugated with Pacific Blue, a-CD150 conjugated with PE and a-CD48 conjugated with FITC. The CD150⁺ CD48- LSK cells were isolated and stained with Vybrant DyD (Invitrogen). A number of HSCs between 5,000 and 11,000 were injected by i.v. route in lethally irradiated *Nes-Gfp* transgenic mice, as described in previous studies (Lo Celso et al., 2009. Nature, 457: 92-96).

### EXAMPLE 6. Determination of the expression and regulation of HSC maintenance genes by Nes:GFP⁺ cells

In order to study in more depth the regulation of the haematopoietic stem cell niche by the G-CSF factor and the Central Nervous System (SNS), we analysed the expression of genes that regulate the maintenance of HSCs and their attraction in the bone marrow (CXCL12/SDF-1, c-kit ligand, angiopoietin-1, interleukin-7, vascular cell adhesion molecule-1, osteopontin and N-cadherin) in CD45⁻ Nes:GFP⁺ and CD45⁻ Nes:GFP- cells isolated from the bone marrow of mice treated with G-CSF or injected with a vehicle, as in previous studies (Katayama et al., 2006. Cell, 124: 407-421). The expression of these genes was extremely high (close to or greater than that of *Gapdh)* in the CD45⁻ Nes:GFP⁺ cells and, with the exception of *Angpt1,* 50 to 800 times greater than in the CD45⁻ Nes:GFP- bone marrow cells. Moreover, the expression of these genes (except for osteopontin and N-cadherin) significantly decreased in the CD45⁻ Nes:GFP⁺ cells, but not in the CD45⁻ Nes:GFP- cells, after the mobilisation of HSCs induced by G-CSF (Fig. 9A) or stimulation of the β₃ adrenergic receptor (Fig. 9B). The use of a different housekeeping gene showed very similar results (Fig. 10). The expression of connexins 45 and 43 was also 200 to 500 times greater in the CD45⁻ Nes:GFP⁺ cells than in the CD45⁻ Nes:GFP- cells (Fig. 9C), which suggests that there is electromechanical coupling between the CD45⁻ Nes:GFP⁺ cells innervated by sympathetic nerve terminals (Katayama et al., 2006. Cell, 124: 407-421; Mendez-Ferrer et al., 2008. Nature, 452: 442-447; Yamazaki and Allen, 1990. Am J Anat, 187: 261-276). Therefore, cytokines, hormones and the SNS regulate both the attraction of HSCs and bone formation in the bone marrow niche by means of direct control of the nestin⁺ MSCs, where the expression of critical genes for the maintenance of HSCs and the cellular fate (proliferation and differentiation) is regulated in a co-ordinated manner.

### Conclusions about the function of nestin⁺ MSC cells in the haemopoietic niche

The relevant role of nestin⁺ MSCs in the haemopoietic niche is based on the following evidence: i) The present invention has shown a marked, nonrandom proximity between both stem cells under homeostasis conditions. This physical association takes place both in the endostium and the medullary parenchyma. ii) Nestin⁺ MSCs are innervated by the SNS, rich in the expression of Cxcl12 and the functional β₃ adrenergic receptor. iii) Nestin⁺ MSCs present very high expression levels of molecules that are critical for the maintenance and the quiescence of HSCs in the "osteoblastic niche", such as angiopoietin-1, osteopontin, N-cadherin and c-kit ligand.

The present invention shows evidence that both the osteoblast proliferation and differentiation of nestin⁺ bone marrow MSCs are selectively induced by PTH and inhibited by G-CSF and the sympathetic fibres that innervate nestin⁺ cells. Therefore, these results extend the previous observations about the inhibitory effect of the SNS on bone formation and the expansion of HSCs induced by PTH (Adams et al., 2007. Nat Biotechnol, 25: 238-243; Calvi et al., 2003. Nature, 425: 841-846), both described as being exclusively mediated by OBs, to MSCs. iv) G-CSF or the agonists of β₃ adrenergic receptors, the activation whereof also promotes the exit of HSCs from the bone marrow, significantly reduced the expression of genes that control the attraction of HSCs in the bone marrow niche (*Cxcl12, Kitl, Angpt1* and *Vcam1).* The results shown in the present invention indicate that the co-ordinated regulation of the haemopoietic and mesenchymal lineages also takes place at the level of stem cells, since neuronal (SNS) and humoral (PTH) homeostatic mechanisms regulate in tandem the maintenance and quiescence of HSCs and the proliferation and differentiation of MSCs. v) It is worth noting that the selective cellular depletion experiments based on two independent transgenic lines (*iDTR* mice crossed with *Nes-Cre* and *Nes-Cre^{ERT2}* animals) resulted, in both cases, in a rapid reduction in the number of CD150⁺ CD48⁻ bone marrow LSK cells and the mobilisation thereof towards the spleen. The fact that the depletion of nestin⁺ cells in primary bone marrow cultures also reduced the number of haemopoietic progenitors demonstrates that the effect was directly caused by selective cell ablation in the bone marrow. On the other hand, this reduction is sharper and more marked than that described following the selective elimination of OBs using the expression of thymidine kinase directed by Col2.3 (Zhu et al., 2007. Blood, 109: 3706-3712), which supports the role of nestin⁺ cells in the haemopoietic niche. vi) Finally, it was demonstrated that nestin⁺ cells are necessary for the nesting of HSCs in the bone marrow using two different methods. In a group of experiments, the elimination of nestin⁺ cells, using both DTR-based depletion models, compromised the nesting of haemopoietic progenitors in the bone marrow of lethally irradiated recipient animals. The same effect was not observed in the migration of leukocytes, in agreement with recent evidence that supports the existence of different mechanisms that regulate the traffic of mature vs. immature blood cells. In another group of experiments that the isolated HSCs with a high degree of purity, labelled and injected in lethally irradiated recipient animals, quickly (in less than two hours) nest close to the Nes:GFP⁺ bone marrow cells, as additional evidence of the role of nestin⁺ MSCs as cells that regulate the attraction of HSCs in the bone marrow niche. Overall, these studies indicate a relevant role for nestin⁺ MSCs in the haemopoietic niche.

The studies performed make it possible to propose the existence of a single niche in the bone marrow formed by the MSC-HSC association, which is strictly regulated at the local level by the microenvironment and also at a distance, by means of humoral and autonomous nervous system signals.

### EXAMPLE 7. Materials and methods

### 7.1. Animals

The following mice were used: *Nes-Gfp* (Mignone et al., 2004. Cell Cycle, 6: 2161-2170), FVB-Tg(*Col1a1-cre*) 1Kry/Mmcd (Dacquin et al., 2002. Dev Dyn, 224: 245-251), B6.Cg(SJL)-TgN(*Nes-cre*)1Kln (Tronche et al., 1999. Nat Genet, 23: 99-103), *Nes-Cre^{ERT2}*, C57BL/6-Gt(ROSA)26Sortm1(HBEGF)Awai/J (Buch et al., 2005. Nat Methods, 2: 419-426), B6;129-Gt(ROSA)26Sortm2Sho/J (Mao et al., 2001. Blood, 97: 324-326), *RCE:LoxP*, Tg(CAG-Bgeo/GFP)21 Lbe/J (*Z*/EG) and B6.12954-Gt(*ROSA*)26Sortm1Sor/J (Jackson Laboratories) transgenic mice and C57BL/6-CD45.1 congenic mice (Frederick Cancer Research Center). Except as otherwise indicated at the foot of the figures, all the animals used were adults. The procedures were approved by the animal use and care committee at the Mount Sinai School of Medicine.

### 7.2. Cell extraction and culture

In order to extract the cells, the bone marrow was eluted with FACS medium described in previous studies (Molofsky et al., 2003. Nature, 425: 962-967), made up of Leibovitz L-15 medium (Invitrogen) supplemented with 1 mg/ml bovine serum albumin (BSA, Sigma), 10 mM HEPES (Sigma), pH 7.4, and 1% penicillin-streptomycin (PS, Invitrogen). After lysing the erythrocytes with 0.8% NH₄Cl, the bone marrow was enzymatically processed in the same manner described to isolate the neural crest cells from the postnatal intestine (Molofsky et al., 2003. Nature, 425: 962-967). The cells were immunomagnetically enriched using magnetic spheres conjugated with an anti-CD45 antibody (Milteyi Biotec), following the manufacturer's instructions, and the CD45⁻ GFP⁺ and CD45⁻ GFP- cells were isolated by means of FACS. For the sphere formation assay, the cells were seeded at clonal density (< 1,000 cells/cm²) in 35-mm plates (*StemCell Technologies*) or in single wells of ultralow-attachment 96-well plates (*Corning*). The composition of the culture medium was adapted from that of neural crest cells (Pardal et al., 2007. Cell, 131: 364-377) and of pericytes (Crisan et al., 2008a. Curr Protoc Stem Cell Biol, Chapter 2, Unit 2B 2 1-2B 2 13), and contained 15% of chicken embryo extract, prepared as previously described (Stemple and Anderson, 1992. Cell, 71: 973-985), 0.1 mM β-mercaptoethanol, 1% of non-essential amino acids (Sigma), 1% of N2 supplement and 2% of B27 supplement (Invitrogen), fibroblast growth factor (FGF)-basic, insulin-like growth factor-1 (IGF-1), epidermal growth factor (EGF), platelet-derived growth factor (PDGF), oncostatin M (OSM) (Peprotech) and leukemia inhibitory factor (ESGRO®, Millipore) (all at 20 ng/ml) in DMEM/F12 (1:1) / human endothelial (Invitrogen) (1:2). The cultures were kept at 37°C with 5% CO₂ in a water bath incubator and left untouched for a week, in order to prevent cell aggregation. Half of the culture medium was changed on a weekly basis.

### 7.3. In vitro differentiation

Osteoblast differentiation was induced by culturing the cells for 4 weeks with 50 µg/ml L-ascorbic acid 2-phosphate, 10 mM glycerophosphate (Sigma) and 15% FBS in α-MEM supplemented with PS (Invitrogen). Adipocyte differentiation was induced with 1 µM dexamethasone, 100 µM indomethacin, 0.5 mM 3-isobutyl-I-methylxanthine (IBMX), 10 µg/ml insulin (Sigma) and 10% FBS in α-MEM supplemented with PS. Chondrocyte differentiation was induced in confluent CFU-F cultures prepared as previously described (Mendez-Ferrer et al., 2008. Nature, 452: 442-447), and also in cell pellets, with 10⁻⁷ M dexamethasone, 10⁻⁴ M L-ascorbic acid, 1 mM sodium pyruvate, 1 mM non-essential amino acids, 1x ITS+1 (10 mg/l bovine insulin, 5.5 mg/l transferrin, 5 µg/L sodium selenite, 4.7 µg/ml linoleic acid, and 0.5 mg/ml bovine serum albumin; Sigma) and 10 ng/ml TGF-β3 (Peprotech) in DMEM (Invitrogen) for 4 weeks. All the cultures were kept at 37°C with 5% CO₂ in a water bath incubator, and half of the medium was changed twice a week. In order to study the differentiation into mesenchymal lineages, the cells were washed with PBS supplemented with 0.1 mM CaCl₂ and 1 mM MgCl₂ (modPBS), and fixed for 10 min at room temperature with 3% paraformaldehyde (Sigma) in modPBS. In order to detect the alkaline phosphatase activity, the cells were washed twice with PBS and incubated for 20 min at room temperature with 50 µg/ml Naphtol AS-MX phosphate, 0.5% N,N-dimethylformamide and 0.6 mg/ml Fast Red Violet LB in 0.1 M Tris-HCl, pH 8.9. The formation of calcium deposits was examined by means of von Kossa staining. To this end, the cells were washed 3 times and stained with newly prepared 5% silver nitrate for 30 min. After 3 washes, the reaction was developed with 5% sodium carbonate in 25% formalin for 5 min. After washing 3 times, the cells were fixed with 5% sodium thiosulfate for 2 min and washed 3 times. The adipocytes were stained with Oil Red O in the following manner: the cells were washed with 60% isopropanol and allowed to dry completely. A 6:4 dilution in distilled water was prepared from a stock solution containing 0.35 g/ml Oil Red O in isopropanol (Sigma), filtered 20 min later. The cells were incubated for 10 min with this solution and washed 4 times. In order to stain the mucopolysaccharides characteristic of the cartilage, the fixed cell cultures and pellets were incubated for 30 min at room temperature with 1% Alcian blue 8GX (Sigma) in 3% acetic acid, pH 2.5, and washed 4 times.

### 7.4. In vivo transplantation

The porous ceramic cubes (∼3 mm³), containing 65% of calcium phosphate hydroxyapatite and 35% of tricalcium phosphate (Ceraform®), were washed twice in order to eliminate small fragments detached from the cubes, autoclaved and coated with 0.1 mg/ml bovine plasma fibronectin (Sigma). In order to eliminate the air from the ossicles and ensure total coating of the surface thereof, the ossicles were placed in a tube containing the fibronectin solution and stirred for 1 min whilst negative pressure was applied by suction with a 60-ml syringe with a 21 g needle through the tube cap. The cap was replaced with a new one and the same process was repeated. The fibronectin-coated ossicles were allowed to dry overnight in a laminar flow hood. The newly isolated cells were introduced into the ossicles by means of the same process described, whereas the spheres were deposited on the surface of the ossicles. In both cases, the cells were allowed to adhere to the ossicles, in the sphere growth medium, for 24 h in the incubator. The ossicles were implanted s.c. under the dorsal skin of anaesthesised adult animals, from the same litter, but which did not have the transgenes.

### 7.5. Histological analyses

Two months after the transplant, the anaesthesised animals were injected with 100 U of sodium heparin (i.p., Sigma), in order to prevent coagulation, and perfused through the left ventricle with ∼ 20 ml of 2 mM MgCl₂ in cold PBS, followed by 100 ml of 2% paraformaldehyde, 0.2% glutaraldehyde, 5 mM EGTA and 2 mM MgCl₂ in PBS, pH 7.4, at 4°C. The ossicles were recovered and post-fixed for 2 h at 4°C with the same fixing solution. Following two washes with 2 mM MgCl₂ in cold PBS, the ossicles were washed for 10 min with 2 mM MgCl₂, 0.01% sodium deoxycholate (Sigma) and 0.02% Nonidet P-40 (Roche) in PBS. The X-gal staining was performed with 5 mM K₃Fe(CN)₆, 5 mM K₄Fe(CN)₆, 2 mM MgCl₂, 0.01 % sodium deoxycholate (Sigma), 1 mg/ml 5-Bromo-4-chloro-3-indoxyl-beta-D-galactopyranoside (X-gal, BioSynth AG®) and 0.02% Nonidet P-40 (Roche) in PBS at 37°C under movement overnight. The ossicles were partially decalcified with 0.25 mM EDTA for 2 or 3 days and processed for cryostat sectioning (10 µm) using a tungsten carbide razor blade (Diamond Knives) and 4x methacrylate-coated plates and the CryoJane adhesive transfer tape system (Instrumedics). The immunohistochemistry with signal amplification was performed as previously described (Mendez-Ferrer et al., 2008. Nature, 452: 442-447). The immunohistochemistry for the detection of the SLAM markers has been described in previous studies (Kiel et al., 2005. Cell, 121: 1109-1121).

### 7.6. In vivo treatment

Isoproterenol or the BRL37344 agonist (2 mg/kg; Sigma) were injected by i.p. route 2 h before sacrificing the animals; the adrenergic agonists were also present, at a concentration of 50 µM, during the enzymatic digestion and cell separation by FACS, performed at 37°C and room temperature, respectively. The treatments with G-CSF (Katayama et al., 2006. Cell, 124: 407-421) or PTH (Adams et al., 2007. Nat Biotechnol, 25: 238-243) have been described in previous studies.

### 7.6. RNA extraction and quantitative PCR

The cells separated by FACS were directly recovered in lysis solution and the RNA was extracted using the Dynabeads® mRNA DIRECT™ Micro Kit (Invitrogen). The reverse transcription was performed with the *Reverse Transcription System* (Promega). The quantitative PCR protocol has been previously described (Mendez-Ferrer et al., 2008. Nature, 452: 442-447). The oligonucleotide sequences used are included in Table 2.

**Table 2. Primer sequences used for the quantitative PCR**

| **Primers** | **Sequence** | **Product (bp)** | **Ring (°C)** | **formation** |
|---|---|---|---|---|
| *Cxc*/*12*_Fw | SEQ ID NO: 1 | | | |
| *Cxc*/*12*_Rv | SEQ ID NO: 2 | 118 | 60 | |
| *Gfp*_Fw | SEQ ID NO: 3 | | | |
| *Gfp*_Rv | SEQ ID NO: 4 | 280 | 60 | |
| *Kitl*_Fw | SEQ ID NO: 5 | | | |
| *Kitl*_Rv | SEQ ID NO: 6 | 65 | 60 | |
| *Angpt1*_Fw | SEQ ID NO: 7 | | | |
| *Angpt1*_Rv | SEQ ID NO: 8 | 138 | 60 | |
| *IL7*_Fw | SEQ ID NO: 9 | | | |
| *IL7*_Rv | SEQ ID NO: 10 | 102 | 60 | |
| *Vcam1*_Fw | SEQ ID NO: 11 | | | |
| *Vcam1*_Rv | SEQ ID NO: 12 | 121 | 60 | |
| *Spp1*_Fw | SEQ ID NO: 13 | | | |
| *Spp1*_Rv | SEQ ID NO: 14 | 149 | 60 | |
| *Ogn*_Fw | SEQ ID NO: 15 | | | |
| *Ogn_*Rv | SEQ ID NO: 16 | 122 | 60 | |
| *Alpl*_Fw | SEQ ID NO: 17 | | | |
| *Alpl*_Rv | SEQ ID NO: 18 | 73 | 60 | |
| *Gpnmb*_Fw | SEQ ID NO: 19 | | | |
| *Gpnmb*_Rv | SEQ ID NO: 20 | 65 | 60 | |
| *Runx2*_Fw | SEQ ID NO: 21 | | | |
| *Runx2*_Rv | SEQ ID NO: 22 | 128 | 60 | |
| *Sp7*_Fw | SEQ ID NO: 23 | | | |
| *Sp7*_Rv | SEQ ID NO: 24 | 275 | 60 | |
| *Bglap*_Fw | SEQ ID NO: 25 | | | |
| *Bglap*_Rv | SEQ ID NO: 26 | 183 | 60 | |
| *Bmp4*_Fw | SEQ ID NO: 27 | | | |
| *Bmp4*_Rv | SEQ ID NO: 28 | 69 | 60 | |
| *Pparg*_Fw | SEQ ID NO: 29 | | | |
| *Pparg_Rv* | SEQ ID NO: 30 | 100 | 60 | |
| Cfd_Fw | SEQ ID NO: 31 | | | |
| Cfd_Rv | SEQ ID NO: 32 | 50 | 60 | |
| Acan_Fw | SEQ ID NO: 33 | | | |
| Acan_Rv | SEQ ID NO: 34 | 269 | 60 | |
| Sox9_Fw | SEQ ID NO: 35 | | | |
| Sox9_Rv | SEQ ID NO: 36 | 186 | 60 | |
| *Gjc1_Fw* | SEQ ID NO: 37 | | | |
| *Gjc1_Rv* | SEQ ID NO: 38 | 245 | 60 | |
| *Gja1_Fw* | SEQ ID NO: 39 | | | |
| *Gja1_Rv* | SEQ ID NO: 40 | 119 | 60 | |
| *Gapdh*_Fw | SEQ ID NO: 41 | | | |
| *Gapdh*_Rv | SEQ ID NO: 42 | 77 | 60 | |

| | | | | |
|---|---|---|---|---|
| Note: The abbreviation Fw refers to primers with forward sequences and the abbreviation Rv refers to primers with reverse sequences. The size of the fragment that is amplified with each of the corresponding Fw/Rv primer pairs is measured in base pairs (pb). The "ring formation" parameter indicates the optimal hybridisation temperature between the Fw and Rv primers and the DNA template sequence. | | | | |

### EXAMPLE 8. Assays with human bone marrow biopsies

### 8.1. Cell extraction and culture

Medullary aspirates obtained from healthy donours were washed with PBS. The bone marrow cells were obtained by centrifugation. The red blood series cells were lysed and the samples were enzymaticaly and mechanically digested as described in the murine samples.

The samples were immunomagnetically depleted from most of the CD45+ cells using magnetic spheres conjugated with an anti-human antibody (Miltenyi Biotec), following the manufacturer's recommendations. The CD45-cells were isolated by means of an automatic separator (FACS) and seeded at clonal density (< 1,000 cells/cm²) under the same conditions that promote the growth of murine mesenspheres, except that the growth factors were of human origin.

The spheres had the same appearance as the murine spheres formed in 7-10 days (Fig. 11).

The primary spheres were individually collected, digested with collagenase (Stem Cell Technologies) and seeded in three different culture media:
1- Mesensphere-forming medium (medium B).
2- Modified mesensphere-forming medium, wherein the chicken embryo extract is replaced with 10% human serum (medium A).
3- Phenol-free αMEM medium, containing 10% FBS and 1% penicillin-streptomycin (Invitrogen), conditions which promote the growth of the fibroblast colony-forming units (CFU-F).

The number of secondary spheres and CFU-F obtained from each primary sphere are indicated in Table 3.

**Table 3. Number of secondary spheres and CFU-F generated from each primary sphere**

| **Medium A** | **Medium B** |
|---|---|
| 43.5 ± 6.6 spheres (n = 24) | 7.4 ± 2.1 spheres (n = 16) |

We observe a significant expansion of the sphere-forming cells following the *in vitro* subculture. The MSC function of the primary spheres was confirmed by the generation of a high number of CFU-F from each primary sphere (Table 3). These CFU-F were alkaline phosphatase+ and were capable of differentiating into adipocytes and mineralising osteoblasts, as was verified by means of von Kossa staining of the calcium deposits (Fig. 12).

In order to test self-renewal *in vitro,* the secondary spheres were digested and subcultured under the same conditions. A mean of 436.4 ± 42.3 tertiary spheres were obtained from each secondary sphere (n = 13). Therefore, these data demonstrated a 3,230-to18,983-fold expansion of the sphere-forming cells. This expansion was approximately 6 times greater in medium A, which had no animal products. Said culture conditions would be potentially useful to expand human mesenchymal stem cells (MSCs).

In order to test self-renewal *in vivo,* the isolated primary spheres were bound to phosphocalcic ceramic ossicles and subcutaneously implanted in immunodeficient mice. The ossicles were extracted after two months, enzymatically digested and separated by FACS using combinations of the MSC CD105, CD140b and CD146 markers, and subcultured in a mesensphere-forming medium. The mesensphere-forming capacity of each population is indicated in Table 4.

**Table 4. Self-renewal of human mesenspheres in vivo. The sphere formation efficiency (as a percentage with respect to the number of cells seeded) of each population within the different ossicles is indicated.**

| **CD105+ CD140b+** | **CD146+ CD140b+** |
|---|---|
| 32 (9/28) | 47 (65/137) |
| 11 (4/38) | 5.5 (1/18) |
| | 10.5 (4/38) |

Despite the low number of mesenspheres that were positive for the classic MSC markers, they were rich in sphere-forming cells, which indicates that human mesenspheres may self-renew *in vivo.*

Our previous studies suggest that Nestin-positive MSCs are required for the self-renewal of HSC. In order to test the possible use of human mesenspheres to promote self-renewal *in vitro* and/or expansion, the human primary mesenspheres formed after 10 days under culture were co-cultured with CD34+ bone marrow cells in serum-free medium (StemSpan, StemCell Technologies), supplemented with 10 mg/ml heparin (Sigma), 10 ng/ml mouse stem cell factor, 20 ng/ml of human thrombopoietin and 10 ng/ml of human FGF-1 (R&D). The presence of mesenspheres in the culture medium was associated with a 21-fold expansion in the number of CD34+ cells after 12 days of culture, as compared to the growth in the absence of mesenspheres under the same conditions. Therefore, the MSCs cultured under these conditions could potentially be used to promote the expansion and self-renewal of HSC cells *in vitro.*

## Claims

1. An isolated cell population which comprises at least one multipotential Nestin-positive stem cell.

2. An isolated cell population according to the preceding claim, wherein the multipotential Nestin-positive stem cell is a mesenchymal stem cell.

3. An isolated cell population according to the preceding claim, wherein the Nestin-positive mesenchymal stem cell is non-adherent.

4. An isolated cell population according to any of claims 1-3, wherein the multipotential Nestin-positive stem cell is obtained by means of a process which comprises:
a) obtaining an aspirate from the bone marrow of a mammal,
b) lysing the red-series cells,
c) selecting the CD45- cells, and
d) seeding the cells of step (c) in an adequate medium.

5. An isolated cell population according to claim 4, wherein the mammal is a human.

6. An isolated cell population according to any of claims 1-5, which further comprises at least one haematopoietic stem cell.

7. An isolated cell population according to claim 6, wherein the haematopoietic stem cell is human.

8. A composition which comprises an isolated cell population according to any of claims 1-7.

9. A composition according to the preceding claim, which is a pharmaceutical composition.

10. A composition according to any of claims 8-9, which further comprises a pharmaceutically acceptable vehicle.

11. A composition according to any of claims 8-10, which further comprises another active principle.

12. The use of an isolated cell population according to any of claims 1-7, or a pharmaceutical composition according to claims 8-11, for the maintenance of haematopoiesis *in vitro.*

13. The use of an isolated cell population according to any of claims 1-7, or a pharmaceutical composition according to claims 8-11, for the self-renewal of haematopoietic stem cells.

14. The use of an isolated cell population according to any of claims 1-7, or a pharmaceutical composition according to claims 8-11, for the expansion of haematopoietic stem cells.

15. The use of an isolated cell population according to any of claims 1-7, or a pharmaceutical composition according to claims 8-11, for the preparation of a medicament.

16. The use of an isolated cell population according to any of claims 1-7, or a pharmaceutical composition according to claims 7-11, for the preparation of a medicament designed for the maintenance of haematopoiesis in a mammal.

17. The use of an isolated cell population or a pharmaceutical composition according to the preceding claim, wherein the mammal is a human.

18. The use of an isolated cell population according to any of claims 1-7, or a pharmaceutical composition according to claims 8-11, for the preparation of a medicament designed for tissue repair and regeneration.

19. The use of an isolated cell population or a pharmaceutical composition according to the preceding claim, wherein the tissue is the blood.

20. The use of an isolated cell population according to any of claims 1-7, or a pharmaceutical composition according to claims 8-11, for the preparation of a medicament designed for the treatment of blood and haematopoietic organ diseases.

21. The use of an isolated cell population or a pharmaceutical composition according to the preceding claim, wherein the disease evolves with a myelopoiesis or lymphopoiesis deficiency.

22. The use of an isolated cell population or a pharmaceutical composition according to any of claims 20-21, wherein the disease is selected from the list that comprises: myeloma, benign monoclonal gammopathy, hypoplasia and medullary aplasia, myelofibrosis, myelodysplastic syndrome, anaemia, polycythaemia, neutropenia, acute leukemia, chronic leukemia, lymphoma, purpura, haemophilia, or any combination thereof.

23. A method for the maintenance of haematopoiesis *in vitro,* which comprises:
a) placing at least one isolated Nestin-positive cell in contact with at least one isolated haematopoietic stem cell, and
b) incubating the product obtained in section (a) in an adequate culture medium for the division and/or differentiation of the haematopoietic stem cell.

24. A method for obtaining haematopoietic cells *in vitro,* which comprises:
a) placing at least one isolated Nestin-positive cell in contact with at least one isolated haematopoietic stem cell, and
b) incubating the product obtained in section (a) in an adequate culture medium for the division and/or differentiation of the haematopoietic stem cell.

25. A method according to any of claims 23-24, wherein the Nestin-positive cell is isolated from the bone marrow.

26. A method according to any of claims 23-25, wherein the Nestin-positive cell is a multipotent stem cell.

27. A method according to any of claims 23-26, wherein the Nestin-positive cell is a mesenchymal stem cell.

28. A method according to any of claims 23-26, wherein the Nestin-positive cell and the haematopoietic stem cell are human.

29. The haematopoietic cells obtainable by the method of claims 24-28.

30. A method for determining the maintenance of the haematopoietic capacity of a mammal, which comprises:
a) analysing at least one expression product of the gene that encodes the Nestin protein in the cells of a sample of haematopoietic tissue isolated from said mammal, and
b) assigning the presence of said expression product analysed in section (a) to the maintenance of the haematopoietic capacity of the organ wherefrom said tissue is derived.

31. A method for determining the maintenance of the haematopoietic capacity of a mammal, which comprises:
a) determining the concentration of at least one expression product of the gene that encodes the Nestin protein in the cells of a sample of haematopoietic tissue isolated from said mammal,
b) calculating the difference between the concentration of the expression product of the gene that encodes the Nestin protein determined in section (a) and the concentration of the expression of said gen in a positive and/or negative control, and
c) assigning a significant difference with respect to the positive control, and/or the absence of a significant difference with respect to the negative control, calculated in section (b), to the maintenance of the haematopoietic capacity of the tissue wherefrom said sample is derived.

32. A method according to any of claims 30 or 31, where the mammal is a human.
